(19) Europäisches Patentamt
European Patent Office
Office européen des brevets

(11) Veröffentlichungsnummer: **0 270 114**
**A2**

# (12) EUROPÄISCHE PATENTANMELDUNG

(21) Anmeldenummer: **87117899.2**

(22) Anmeldetag: **03.12.87**

(51) Int. Cl.⁴: **C12N 15/00**, C12P 21/00, C07K 15/00, G01N 33/569, A61K 39/21, G01N 33/68

Patentanspruch für folgenden Vertragsstaat: ES.

Der (Die) Mikroorganismus (Mikroorganismen) ist (sind) bei Deutsche Sammlung von Mikroorganismen unter der (den) Nummer(n) 3516, 3517, 3519 hinterlegt worden.

(30) Priorität: **05.12.86 GB 8629116**

(43) Veröffentlichungstag der Anmeldung: **08.06.88 Patentblatt 88/23**

(84) Benannte Vertragsstaaten: **BE CH DE ES FR GB IT LI NL**

(71) Anmelder: **F. HOFFMANN-LA ROCHE & CO. Aktiengesellschaft**

**CH-4002 Basel(CH)**

(72) Erfinder: **Gentz, Reiner, Dr.**
**Am Hochgericht 34**
**D-7888 Rheinfelden(DE)**
Erfinder: **Le Grice, Stuart**
**Spalenring 63**
**CH-4055 Basel(CH)**
Erfinder: **Mous, Jan, Dr.**
**Liebrütistrasse 15**
**CH-4303 Kaiseraugst(CH)**
Erfinder: **Stüber, Dietrich, Dr.**
**Dürerstrasse 41**
**D-7888 Rheinfelden(DE)**

(74) Vertreter: **Lederer, Franz, Dr. et al**
**Van der Werth, Lederer & Riederer**
**Patentanwälte Lucile-Grahn-Strasse 22**
**D-8000 München 80(DE)**

## (54) ENV/GAG-Polypeptide.

(57) env-gag-Polypeptide und deren Herstellung mittels rekombinanter DNA-Technologie, sowie die in diesem Verfahren verwendeten Gene, Expressionsvektoren und transformierten Mikroorganismen werden beschrieben. Ferner werden Verfahren zum Nachweis von AIDS-Antikörpern oder AIDS-Viren in menschlichen Seren oder anderen biologischen Flüssigkeiten sowie Methoden zum immunoprophylaktischen Schutz der Menschen auf der Basis dieser env/gag-Polypeptide beschrieben. Schliesslich wird ein lange Zeit stabiles Test-Kit zur Bestimmung von AIDS-Antikörpern in menschlichen Seren beschrieben.

Fig. 8

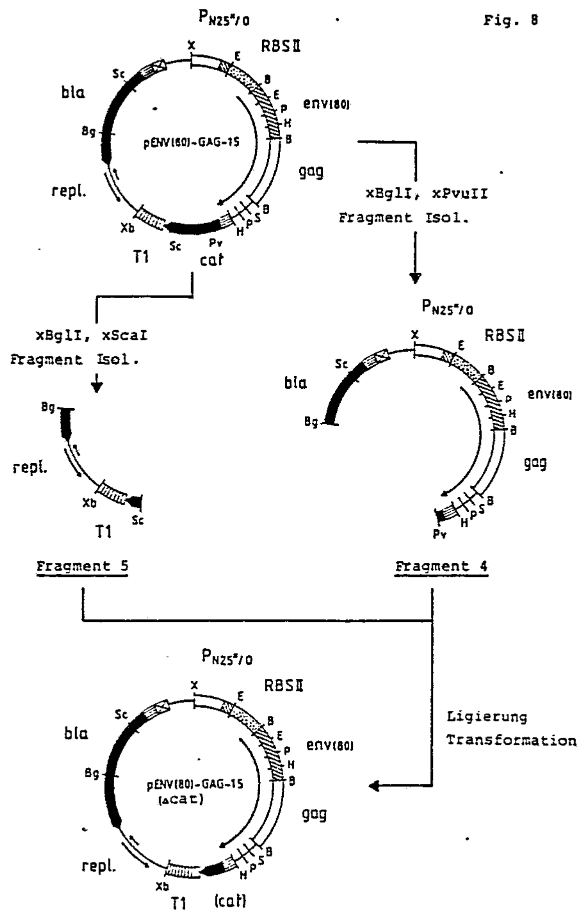
pENV(80)-GAG-15
xBglI, xPvuII
Fragment Isol.
xBglI, xScaI
Fragment Isol.
Fragment 5
Fragment 4
Ligierung
Transformation
pENV(80)-GAG-15
(Δcat)

## ENV/GAG-Polypeptide

Die vorliegende Erfindung betrifft Polypeptide mit der Bezeichnung env/gag, welche determinante Gruppen, die mit den Sequenzen eines env und gag Proteins des verursachenden Agenz von AIDS (acquired immune deficiency syndrome) übereinstimmen, einschliessen, Expressionsvektoren, welche die env/gag-Polypeptide kodieren, die Herstellung der env/gag-Polypeptide mittels rekombinanter DNA-Technologie, Verfahren zum Nachweis von AIDS-Antikörpern oder AIDS-Viren in menschlichen Seren oder anderen biologischen Körperflüssigkeiten sowie Verfahren zum immunoprophylaktischen Schutz der Menschen vor AIDS auf der Basis der env/gag-Polypeptide. Die Erfindung betrifft ferner ein lange Zeit stabiles Test-Kit zur Bestimmung von AIDS-Antikörpern in menschlichen Seren.

1985 wurden nahezu 8000 Menschen mit AIDS diagnostiziert und die Zahl ist seither ständig gestiegen. Man rechnet mit weiteren fünfzehntausend Fällen im Jahre 1986 und es ist nicht ausgeschlossen, dass sich die Zahl der Fälle im Jahre 1987 erneut verdoppelt [New York Times Magazine, 2. März 1986, S. 42]. AIDS wird charakterisiert durch das Auftreten schwerer opportunistischer Infektionen einhergehend mit einem Angriff auf das Immunsystem des Körpers [Gottlieb et al., New Eng. J. Med. 305, 1426 (1981)]. Die Krankheit wurde bei homosexuellen Männern, Patienten, die Bluttransfusionen erhielten, Drogensüchtigen, die die Drogen intravenös applizieren, und aus Haiti und Zentralafrika stammenden Personen festgestellt [Piot et al., Lancet 11, 65 (1984)].

Man nahm an, dass das auslösende Agenz viralen Ursprungs sei, weil die epidemiologische Verbreitung von AIDS mit der einer übertragbaren Krankheit übereinstimmte. Mindestens drei Retroviren wurden von kultivierten T-Zellen verschiedener AIDS-Patienten oder von weissen Blutzellen krankheitsgefährdeter Personen isoliert. Ferner wurde ein neuer menschlicher Retrovirus mit der Bezeichnung LAV (lymphadenopathy-associated virus) als AIDS verursachendes Agenz entdeckt. Dieser Virus wurde von einem Lymhadenopathie-Patienten isoliert, daher der Name [Montagnier et al., in Human T-Cell Leukemia/Lymphoma Virus, R.C. Gallo et al. eds., Cold Spring Harbor Laboratory, pp. 363-370 (1984)].

Weitere menschliche Retroviren, insbesondere zwei Untergruppen des menschlichen T-Zell Leukämie/Lymphoma/Lymphotropischen Virus, Typ I [Poiesz et al., Proc. Natl. Acad. Sci. U.S.A. 77, 7415 (1980)] und III [Popovic et al., Science 224, 497 (1984)] wurden ebenfalls isoliert. Noch ein weiterer Virus, der ARV-Virus (AIDS-associated retrovirus) wurde als auslösendes Agenz erkannt [Levy et al., Science 225, 840 (1984)]. Sowohl der HTLV-III-als auch der ARV-Retrovirus besitzen biologische und seroepidemiologische Eigenschaften, die mit dem LAV übereinstimmen [Levy et al., supra, Popovic et al., supra]. Folglich wurden mindestens drei Retroviren als verursachende Agenzien von AIDS postuliert: LAV, ARV und HTLV-III. In dieser Anmeldung werden sie kollektiv als AIDS-Viren/Human Immunodeficiency Viruses (HIV) bezeichnet. Da der HTLV-III Virus als Prototyp dieser Virusgruppe gilt, bezieht sich die Bezeichnung "Determinante Gruppen, die mit den Sequenzen von HTLV-III-Virusproteinen übereinstimmen" auf Proteinsequenzen aller drei AIDS-Viren.

Ein Grund für die Schwierigkeiten bei der Bestimmung des tatsächlichen AIDS verursachenden Agenz war die Kreuzreaktion verschiedener retroviraler Antigene mit Serenproben von AIDS-Patienten. Zum Beispiel zeigten Serenproben von AIDS-Patienten Reaktionen mit Antigenen des HTLV-I [Essex et al., Science 220, 859 (1983)] und HTLV-III [Sarngadharan et al., Science 224, 506 (1984)] Virus. Hüllproteingenprodukte von HTLV-III Viren zeigten Kreuzreaktivität mit Antikörpern in Seren von erwachsenen T-Zell Leukämie-Patienten [Kiyokawa et al., Proc. Natl. Acad. Sci. U.S.A. 81, 6202 (1984)]. Erwachsene T-Zell Leukämien (ATL) unterscheiden sich von AIDS darin, dass HTLV-I T-Zell Malignitäten verursacht, die durch ein unkontrolliertes Wachstum von T-Zellen charakterisiert sind. Bei AIDS gibt es statt eines Zellwachstums den Zelltod. Diese cytopathischen Eigenschaften von HTLV-III Viren waren entscheidend bei der endgültigen Bestimmung des spezifischen retroviralen Ursprungs dieser Krankheit.

Das verursachende Agenz von AIDS wurde unter Verwendung von immortalisierten menschlichen Neoplasmen-T-Zell-Linien (HT) isoliert, welche mit für AIDS charakteristischen cytopathischen und von AIDS-Patienten isolierten Retroviren infiziert wurden. Seroepidemiologische Tests, bei denen diese Viren verwendet wurden, zeigten eine vollständige Korrelation zwischen AIDS und dem Vorhandensein von Antikörpern gegen virale HTLV-III Antigene [Montagnier et al., supra; Sarngadharan et al., supra; Schüpbach et al., Science 224, 503 (1984)]. Ausserdem fand man heraus, dass nahezu 85% der Patienten mit Lymphadenopathie Syndrom und ein hervorstechender Anteil asymptomatischer homosexueller Männer in AIDS endemischer Umgebung zirkulierende Antikörper gegen HTLV-III in sich trugen. Zusammengefasst sprechen diese Daten dafür, dass die HTLV-III Viren als hauptsächliche AIDS verursachende Agenzien anzusehen sind.

Bis zur erfolgreichen Züchtung des AIDS-Virus unter Verwendung der H-9 Zell-Linie (PCT Anmeldung,

Publikations-Nr. WO85/04897) konnte das env-Protein des AIDS-Virus weder isoliert, noch charakterisiert oder synthetisiert werden. Dies lag hauptsächlich daran, dass der Virus cytopathisch ist und seine Isolierung deshalb nicht möglich war [Popovic et al., supra]. Sobald jedoch eine menschliche T-Zell-Linie entdeckt wurde, die gegen die cytopathischen Wirkungen des Virus resistent war, konnte die molekulare Klonierung der AIDS proviralen DNA durchgeführt werden.

Das Bedürfnis nach genauen und schnellen Methoden für die Diagnose von AIDS in menschlichem Blut und in anderen biologischen Körperflüssigkeiten und das Bedürfnis nach einer Methode zur Verhinderung der Krankheit durch Impfung ist sehr gross. Alle zur Zeit verfügbaren Testmethoden sind jedoch fehlerhaft. Das CDC (Center for Disease Control) hat sogar darauf hingewiesen, dass die momentan verfügbaren Testmethoden ausschliesslich zur Untersuchung von Blutproben auf das Vorhandensein von Antikörpern gegen HTLV-III verwendet werden sollten. Das CDC ging sogar noch weiter indem es erklärte, dass die zur Zeit verfügbaren ELISA (enzyme-linked immunosorbent assay) Testmethoden nicht für die generelle Untersuchung von Risikogruppen oder als Diagnose-Test für AIDS verwendet werden sollten [Federal Register 50(48), 9909, 12. März 1985].

Bei früher angewendeten AIDS-Testmethoden hat man versäumt, ein spezifisches antigenes Protein des verursachenden Agenz für AIDS zu verwenden. Die zuerst verwendeten Proteine stammten von einem viralen Lysat. Da dieses Lysat aus menschlichen, mit dem Virus infizierten Zellen hergestellt wurde, enthielt es sowohl menschliche, als auch virale Proteine. Die Herstellung eines reinen Antigens aus viralem Protein ist sehr schwierig. Die bisher verwendeten Antigene brachten daher sowohl falsch positive als auch falsch negative Ergebnisse [Budiansky, Natur 312, 583 (1984)]. Die durch die Verwendung solcher Protein/Peptid-Lysate entstandenen Fehler könnten durch die Verwendung einer zur Bindung von AIDS-Antikörpern geeigneten Verbindung, die im wesentlichen frei von Nicht-AIDS spezifischen Proteinen ist, verhindert werden. Verbindungen aus im wesentlichen reinen AIDS Hüll-und Strukturproteinen können als Antigene verwendet werden.

Sowohl das Hüll-als auch das Strukturprotein des HTLV-III Virus besitzen determinante Gruppen, die die Untersuchung und Diagnose auf AIDS-Viren und/oder den Schutz durch Impfung gegen AIDS-Viren ermöglichen würden. Und Personen, die mit HTLV-III Viren in Berührung gekommen sind, und die demzufolge AIDS-gefährdet sind oder die die Krankheit haben, können durch die Existenz von Antikörpern gegen das virale Strukturprotein (gag) und/oder das Hüllprotein (env) identifiziert werden [Sarngadharan et al., Science 224, 506 (1984)].

Die Verfügbarkeit einer wirksamen Testmethode zur Bestimmung des AIDS-Virus oder von Partikeln davon im Blut oder in anderen Körperflüssigkeiten ist ebenfalls wichtig. Groopman et al. [Blood 66, 742 (1985)] haben berichtet, dass Antikörper gegen AIDS-Viren nicht immer im Blut von AIDS-Kranken vorhanden sind. Groopman et al. haben einen Patienten mit AIDS und einen weiteren mit AIDS ähnlicher Krankheit (ARC) untersucht, deren Blutproben Antikörper-negativ waren, von denen aber HTLV-III Viren gezüchtet werden konnten.

Vor kurzem wurde die rekombinante DNA-Technologie auf das AIDS-Problem angewendet. Das LAV, HTLV-III und ARV-II Genom wurde kloniert [Schüpbach et al., Science 224, 503 (1984); Alizon et al., Nature 312, 757 (1984)]. Die vollständige Nukleotidsequenz des LAV, ARV und HTLV-III proviralen Genoms wurde bestimmt [Ratner et al., Nature 313, 277 (1985); Sanchez-Pescador et al., Science 227 , 484 (1985); Wain-Hobson et al., Cell 40, 9 [1985)]. Die Klonierung und Expression des HTLV-III env-Gens wurde von Crowl et al. [Cell 41, 979 (1985)] und von Chang et al. [Biotechnology 3, 905 (1985)] beschrieben. Dowbenko et al. [Proc. Natl. Acad. Sci. U.S.A. 82, 7748 (1985)] haben das HTLV-III Strukturprotein in E. coli exprimiert.

Durch Anwendung solcher Verfahrenstechniken der Gentechnologie erhielt man gereinigte, virale Antigene, die sicher und zuverlässig sind und deren Herstellung weniger kostspielig ist. Noch besser wäre jedoch die Verfügbarkeit eines viralen Proteins mit determinanten Gruppen des env-Proteins und des gag-Proteins. Ein solches Protein wäre ein aussergewöhnlich wirksames Mittel für den Nachweis von AIDS-Antikörpern und könnte als Grundlage für eine Reihe von genauen diagnostischen Tests und für mögliche Impfungen gegen das AIDS-Virus dienen.

Deshalb wurden die Polypeptide der vorliegenden Erfindung durch die Anwendung rekombinanter DNA-Techniken hergestellt. Diese Polypeptide gestatten den Nachweis von AIDS-Viren in menschlichen Seren oder anderen biologischen Körperflüssigkeiten und können als Impfstoff Menschen vor AIDS schützen. Unter AIDS-Viren versteht man alle Varianten, die als AIDS verursachende Agenzien in Frage kommen, wie z.B. LAV, ARV und HTLV-III.

Genauer gesagt, liefert die vorliegende Erfindung ein Polypeptid mit einer Aminosäuresequenz der Formel

```
         5         10        15        20        25        30
         |         |         |         |         |         |
  1 M R G S E A Q Q H L L Q L T V W G I K Q L Q A R I L A V E R
 31 Y L K D Q Q L L G I W G C S G K L I C T T A V P W N A S W S
 61 N K L L E Q I W N N M T W M E W D R E I N N Y T G S A D T G
 91 H S S Q V S Q N Y P I V Q N I Q G Q M V H Q A I S P R T L N
121 A W V K V V E E K A F S P E V I P M F S A L S E G A T P Q D
151 L N T M L N T V G G H Q A A M Q M L K E T I N E E A A E W D
181 R V H P V H A G P I A P G Q M R E P R G S D I A G T T S T L
211 Q E Q I G W M T N N P P I P V G E I Y K R W I I L G L N K I
241 V R M Y S P T S I L D I R Q G P K E P F R D Y V D R F Y K T
271 L R A E Q A R I R R P A A K L G E I F R S (ENV(80)-GAG-15)
```

oder Fragmente davon oder Analoga, die sich von besagtem Polypeptid und besagten Fragmenten durch Aminosäuresubstitution(en) unterscheiden, die mindestens eine antigene und/oder immunogene determinante Gruppe des HTLV-III env-Proteins und des HTLV-III gag-Proteins aufweisen (im folgenden env/gag-Polypeptide).

Aminosäuresubstitutionen, die die biologische Aktivität von Proteinen nur unwesentlich verändern, sind an sich bekannt und beispielsweise von H. Neurath und R.L. Hill in "The Proteins", Academic Press, New York (1979), speziell in Fig. 6 auf Seite 14, beschrieben. Die häufigst vorkommenden Aminosäuresubstitutionen sind Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, Asp/Gly und umgekehrt.

Die env/gag-Polypeptide der vorliegenden Erfindung können aufgrund ihrer Herstellungsverfahren Methionin (für das ATG kodiert) als erste N-terminale Aminosäure enthalten. Andererseits kann der mikrobielle Wirt das Translationsprodukt teilweise oder vollständig prozessieren, wodurch das N-terminale Methionin abgespalten wird.

Die env/gag-Polypeptide der vorliegenden Erfindung können aus einem vorstehend besagten env/gag-Polypeptid und einem Trägerprotein, welches sich für die Reinigung, beispielsweise mittels Affinitätschromatographie, besonders eignet, zusammengesetzt sein. Als brauchbare Trägerproteine kommen E. coli Chloramphenicoltransferase (CAT) und Dihydrofolatreduktase (DHFR), speziell der Maus, in Frage.

Die Erfindung betrifft ferner Gene, die die env/gag-Polypeptide der vorliegenden Erfindung kodieren, Expressionsvektoren, die diese Gene enthalten, sowie Transformanten zur Herstellung der env/gag-Polypeptide der vorliegenden Erfindung mittels rekombinanter DNA-Technologie. Es werden auch Methoden zur Expression, Isolierung und Reinigung der env/gag-Polypeptide der vorliegenden Erfindung beschrieben. Die so hergestellten env/gag-Polypeptide können mit den Methoden dieser Erfindung für eine Anzahl wichtiger immunologischer Prozesse verwendet werden.

Als diagnostische Reagenzien verwendet können die env/gag-Polypeptide der vorliegenden Erfindung zum Nachweis von AIDS-Antikörpern in menschlichen Seren oder anderen biologischen Flüssigkeiten, wie z.B. in Tränenflüssigkeit, Samen, Vaginalsekret und Speichel, verwendet werden. Da die env/gag-Polypeptide in homogener Form hergestellt werden können, können Probleme mit unspezifischen Reaktionen, die die Verwendung von diagnostischen Reagenzien auf der Basis relativ roher viraler HTLV-III Proteinlysate in der Vergangenheit eingeschränkt haben, eliminiert werden.

Als Immunogen verwendet können die env/gag-Polypeptide der vorliegenden Erfindung zur Produktion von monoklonalen und/oder polyklonalen Antikörpern, die gegen die in diesem Protein enthaltenen antigenen Determinanten gerichtet sind, verwendet werden. Solche Antikörper können wiederum, in Verbindung mit den env/gag-Polypeptiden der vorliegenden Erfindung, die entsprechend markiert wurden, in einem Radio immunassay (RIA) oder in einem Enzymimmunoassay (ELISA) verwendet werden, um die Gegenwart von AIDS-Viren oder Partikeln davon in menschlichen Seren oder in anderen biologischen Flüssigkeiten festzustellen. Die Partikel (oder Fragmente) von AIDS-Viren, die mit diesen Methoden nachgewiesen werden können, umfassen natürliche Teile des viralen Strukturproteins (gag) und/oder Hüllproteins (env).

Die env/gag-Polypeptide der vorliegenden Erfindung können jedoch nicht nur als diagnostische Werkzeuge sondern auch als therapeutische Mittel verwendet werden. Indem man die env/gag-Polypeptide der vorliegenden Erfindung einer passenden Formulierung beifügt, können sie zum immunoprophylaktischen Schutz vor AIDS verwendet werden.

Die vorliegende Erfindung liefert im weiteren ein diagnostisches Verfahren zum Nachweis von AIDS-Antikörpern und ein für dieses Verfahren geeignetes Test-Kit. Dieses diagnostische Verfahren überwindet die Probleme mit unspezifischen Reaktionen aller früher verwendeter AIDS-Tests.

Die Methoden der vorliegenden Erfindung enthalten eine Anzahl von Verfahrensschritten, die in logischer Folge die folgenden Massnahmen umfassen: (1) Herstellung der Gene, die die env-und gag-Sequenzen kodieren, (2) Einschleusen dieser Gene in einen passenden Vektor zur Herstellung eines rekombinanten Vektors, der ein env/gag-Fusionsgen enthält, (3) Transfer des rekombinanten Vektors in einen verträglichen einzelligen Wirtsorganismus, (4) Selektion und Kultivierung von transformierten Wirtszellen, die die eingeschleusten Gensequenzen replizieren und exprimieren können, (5) Identifizierung und Reinigung des Genprodukts, (6) Verwendung des Genproduktes zum Nachweis von Antikörpern gegen HTLV-III Viren oder verwandte Viren oder zur Herstellung von Antikörpern, die wiederum zum Nachweis der Viren selbst oder Fragmenten davon in menschlichen Seren oder in anderen biologischen Flüssigkeiten verwendet werden können, und (7) Verwendung des env/gag-Proteins in einem Impfstoff zum immunoprophylaktischen Schutz vor AIDS.

Isolierte provirale HTLV-III DNA kann sowohl als Quelle für das gag-Gen, als auch für das env-Gen der vorliegenden Erfindung verwendet werden.

Alternativen zur Isolierung der env-und gag-Gensequenzen beinhalten (aber sind nicht beschränkt auf) die chemische Synthese der entsprechenden Gensequenzen und die Herstellung von DNA, die zu den von den entsprechenden Genen hergestellten Boten-RNAs komplementär ist.

Nach der Identifizierung und Isolierung der env-und gag-Gene können diese nach bekannten Methoden in jeden geeigneten, von Plasmiden oder Phagen abstammenden Expressionsvektor eingebaut werden. Geeignete, von Plasmiden oder Phagen abstammende Expressionsvektoren sind beispielsweise in dem Lehrbuch von Maniatis et al. ("Molecular Cloning", Cold Spring Harbor Laboratory, 1982) erwähnt.

Nach erfolgtem Einbau einer der beiden Gensequenzen (env oder gag) in einen geeigneten Vektor, kann die andere Gensequenz mittels wohlüberlegter Spaltung mit einem Restriktionsenzym in die unmittelbare Nachbarschaft der ersten Gensequenz gebracht werden, wodurch ein Fusionsgen entsteht.

In der bevorzugten Ausführungsform der vorliegenden Erfindung wurden das XhoI/BamHI Fragment des Plasmids pENV(80)-DHFR, welches das synthetische Gen env(80) mit einer Coliphagen T5 Promoter/lac-Operator Expressionskontrollsequenz operabel verknüpft enthält, und gag-Sequenzen in einem BamHI Fragment mit XhoI und BamHI gespaltener DNA des Plasmids pDS5/RBSII,3A+5A verbunden, um den Expressionsvektor pENV/(80)-GAG-15, welcher die Synthese des ENV(80)-GAG-15 Polypeptids bewirkt, zu isolieren. Durch Deletion von Teilen des cat-Gens des Plasmids pENV(80)-GAG-15 wurde anschliessend der Expressionsvektor pENV(80)-GAG-15( Δ cat) isoliert, um die Expression des ENV(80)-GAG-15 Polypeptids zu verbessern.

Die Details dieser Konstruktionen sind in den Beispielen 1 bis 7 beschrieben. Die Nukleotidsequenz des ENV(80)-GAG-15 Gens [welche nach der von Sanger et al. in PNAS U.S.A. 74, 5463 (1977) beschriebenen Methode bestimmt wurde] und die davon abgeleitete Aminosäuresequenz des ENV(80)-GAG-15 Polypeptids sind in Figur 9 gezeigt.

E. coli Stämme, welche die für diese Konstruktionen verwendeten Plasmide enthalten (E. coli M15 enthaltend die Plasmide pDS5/RBSII,3A+5A, pDMI, 1 bzw. pDS8/RBSII, pDMI,1 und E. coli TBI enthaltend Plasmid pA-ENV-20) wurden bei der Deutschen Sammlung von Mikroorganismen (DSM) in Göttingen am 3. Oktober 1985 unter den DSM Nrn. 3517, 3519 bzw. 3516 nach dem Budapester Vertrag hinterlegt.

Natürlich können die in Expressionsvektoren eingebauten env-und gag-Gensequenzen mit DNA-Sequenzen fusioniert sein, die nicht Teil der tatsächlichen Strukturgene sind. Andererseits können die in Expressionsvektoren eingebauten env-und gag-Gensequenzen nur einen Teil der vollständigen Gene kodieren. Es ist nur erforderlich, dass die in einen Expressionsvektor eingebauten Gene die Synthese eines Polypeptids [ENV(80)-GAG-15 oder Fragmente davon oder Analoge, die durch Aminosäuresubstitution(en) erhalten wurden] mit mindestens einer antigenen und/oder immunogenen determinanten Gruppe des env-Proteins und des gag-Proteins eines AIDS-Virus bewirken.

Die Methoden zur Expression von DNA-Sequenzen, welche für die env/gag-Polypeptide der vorliegenden Erfindung kodieren, sind an sich bekannt und beispielsweise in dem vorstehend genannten Lehrbuch von Maniatis et al. beschrieben. Sie umfassen die folgenden Massnahmen:

(a) Transformation eines geeigneten Wirtsorganismus mit einem Expressionsvektor, in welchem die besagte DNA-Sequenz mit einer Expressionskontrollsequenz operabel verknüpft ist;

(b) Kultivierung des so erhaltenen Wirtsorganismus unter geeigneten Wachstumsbedingungen; und

(c) Extraktion und Isolierung des gewünschten Proteins.

Der Fachmann kann aus den bekannten Methoden diejenigen auswählen, welche für eine spezielle Genexpression am wirksamsten sind, ohne dadurch vom Inhalt der vorliegenden Erfindung abzuweichen.

Die Auswahl eines geeigneten Wirtsorganismus wird von verschiedenen dem Fachmann bekannten Faktoren bestimmt. So spielen beispielsweise Kompatibilität mit dem ausgewählten Expressionsvektor, Toxizität der Expressionsprodukte, mühelose Reinigung des gewünschten Proteins, Expressionscharakteristika, notwendige biologische Sicherheitsvorkehrungen und Kosten eine Rolle. Innerhalb dieser allgemeinen Richtlinien kommen als geeignete Wirtsorganismen gram-negative und gram-positive Bakterien, vorzugsweise E. coli und B. subtilis Stämme, in Frage. Ein besonders bevorzugter Wirtsorganismus der vorliegenden Erfindung ist der E. coli Stamm M15 (der von M.R. Villarejo et al. in J. Bacteriol. 120, 466 [1974] als Stamm DZ 292 beschrieben wurde). Es können aber auch andere allgemein zugängliche E. coli Stämme, beispielsweise E. coli 294 (ATCC Nr. 31446), E. coli RR1 (ATCC Nr. 31343) und E. coli W3110 (ATCC NR. 27325) verwendet werden.

Nach der Expression in E. coli sind die env/gag-Polypeptide der vorliegenden Erfindung hauptsächlich in cytoplasmatischen Einschlusskörpern (unlösliche Proteinaggregate) zu finden, was die Reinigung dieser Polypeptide erheblich erleichtert. Zur Isolierung der env/gag-Polypeptide der vorliegenden Erfindung werden die Bakterienzellen zerstört oder lysiert und die unlöslichen Polypeptide werden danach mittels Zentrifugation präzipitiert. Eine weitere wesentliche Reinigung kann dann mittels sequentieller Waschungen des Präzipitats mit Puffer, Detergenz und Salzlösungen gefolgt von Standardproteinreinigungsmethoden erreicht werden.

Zur Gewinnung von geringen Mengen an env/gag-Probenmaterial, wie z.B. Proben für analytische Polyacrylamidgelelektrophoresen, können die Zellen mittels eines Detergenz, z.B. Natriumdodecylsulfat (SDS) aufgeschlossen werden. Zur Gewinnung von grossen Proteinmengen können die Zellen mittels Ultraschall, oder mit mechanischen Mitteln, z.B. der French-Druckzelle, aufgeschlossen werden. Vorzugsweise werden die Zellen mit chemischen oder enzymatischen Mitteln, z.B. EDTA oder EGTA und Lysozym lysiert.

Vorzugsweise wird das unlösliche Proteinpräzipitat in drei getrennten Schritten gewaschen, obgleich es auch denkbar ist, dass gewisse Schritte kombiniert, umgeordnet oder eliminiert werden können. Die erste Waschung des Präzipitats kann mittels einer Pufferlösung, vorzugsweise 10 mM Tris, pH 8.0, erfolgen. Die zweite Waschung des Präzipitats erfolgt mittels einer Detergenzlösung, vorzugsweise 0,5% Triton X-114. Die dritte und letzte Waschung erfolgt mittels einer Salzlösung, vorzugsweise 7M Guanidin-HCl.

Die env/gag-Polypeptidpräzipitate, welche nach der letzten Waschung erhalten werden, können dann mittels Standardproteinreinigungsmethoden, wie z.B. Gelfiltration, Chromatographie, präparative isoelektrische Flachbettfokussierung, Gelelektrophorese, hochauflösende Flüssigkeitschromatographie (im folgenden HPLC; einschliesslich Gelfiltrations-Ionenaustauscher-und Umkehrphasen-Chromatogrphie) und Affinitätschromatographie, z.B. mit Farbstoffen, die an Trägermaterialien gebunden sind oder mit an Sepharose gebundenen monoklonalen Antikörpern, die gegen die besagten env/gag-Polypeptide gerichtet sind, weiter gereinigt werden. Vorzugsweise werden die env/gag-Polypeptidpräzipitate mittels Ausschluschromatographie unter Verwendung von Superose 12 oder Sephacryl S-200 weiter gereinigt.

Ausführliche Details der Reinigung des bevorzugten env/gag-Polypeptids der vorliegenden Erfindung (Env(80)-GAG-15) sind in Beispiel 7 beschrieben.

Die env/gag-Polypeptide der vorliegenden Erfindung können zusammen mit einem verträglichen Trägermaterial als Impfstoff verwendet werden. Sie können in gereinigter Form oder in Form von stark immunogen wirkenden Modifikationen, die nach bekannten Methoden erhalten werden, verwendet werden. Beispielsweise, können die env/gag-Polypeptide durch Quervernetzung mittels 1,3-Dicyclohexylcarbodiimid in eine hoch immunogene Matrix umgewandelt werden. Die env/gag-Polypeptide können aber auch direkt oder indirekt über einen geeigneten Linker an eine hoch immunogene Matrix, z.B. Schnecken-Hämocyanin und verschiedene bakterielle Toxoide (inaktive Toxine), wie z.B. Diphteria-Toxin, gekoppelt werden. In den Fällen, in denen die env/gag-Polypeptide an ein bakterielles Toxoid gekoppelt werden, kann ein bivalenter Impfstoff hergestellt werden, der sowohl vor AIDS als auch vor dem Bakterium, von dem das Toxoid herstammt, schützt. Ferner, kann der Impfstoff weitere Antigene enthalten, welche auch vor anderen Krankheiten schützen.

Die Form der Verabreichung, die Antigen Dosierung und die Häufigkeit der Injektionen sind an sich bekannt und in der einschlägigen Literatur beschrieben.

Die eng/gag-Polypeptide der vorliegenden Erfindung können auch als diagnostische Reagenzien zum Nachweis von AIDS-assoziierten Antikörpern in zahlreichen bekannten Nachweisverfahren verwendet werden. Der Hauptvorteil bei der Verwendung der erfindungsgemässen env/gag-Polypeptide zum Nachweis von AIDS-Antikörpern ist in ihrer Spezifität im Vergleich zur Spezifität der in der Vergangenheit verwendeten Antigene zu sehen.

Bei einem dieser Verfahren zum Nachweis von AIDS-Antikörpern kommt die sogenannte "Western-Blotting" Analyse zur Anwendung [Tobin et al., Proc. Nat. Acad. Sci. USA 76, 4350-4354(1979)]. Bei diesem Nachweisverfahren wird ein Polypeptid der vorliegenden Erfindung mittels Elektrophorese aus einem SDS-Polyacrylamidgel auf Nitrocellulosepapier transferiert. Dieses Nitrocellulosepapier wird dann mit dem zu testenden Serum inkubiert. Nach einer Waschung wird es anschliessend mit anti-human-Ig-Peroxidase-Konjugat inkubiert. Die Peroxidase-Aktivität wird dann mittels eines geeigneten Substrats, wie z.B. O-Phenyldiamin, nachgewiesen. Natürlich können auch radioaktive oder fluoreszierende Markierungen verwendet werden.

Ein geeignetes Verfahren zum Nachweis von AIDS-Antikörpern mittels der erfindungsgemässen env/gag-Polypeptide ist der ELISA (enzyme-linked immunosorbent assay) Test. Dieser Test ist dadurch gekennzeichnet, dass man

(a) ein env/gag-Polypeptid der vorliegenden Erfindung an festem Trägermaterial immobilisiert;

(b) eine menschliche Serumprobe, die im Verdacht steht Antikörper gegen AIDS-Viren zu enthalten, mit diesem immobilisierten Polypeptid in Kontakt bringt;

(c) nicht-gebundenes Material durch Waschung entfernt; und

(d) die gewaschenen immobilisierten Polypeptid/Antikörper-Komplexe durch Zugabe eines markierten Reagenz, welches selektiv menschliche Antikörper erkennt, wie z.B. markiertes Staphylococcus aureus Protein A oder anti-human Ig, nachweist.

Als geeignete Trägermaterialien kommen die inneren Wände von Testgefässen (Reagenzgläser, Titerplatten und Küvetten aus Glas oder Kunststoff) sowie die Oberflächen von Festkörpern (Glasstäbe, Kunststoffstäbe, Stäbe mit Verdickungen an den Enden und Stäbe mit Lamellen oder Kugeln an den Enden) in Frage. Als besonders geeignete Trägermaterialien kommen Glas-oder Kunstoffkugeln in Frage.

Als Markierungen eignen sich alle nachweisbaren funktionellen Gruppen, die keinen Einfluss auf die Wechselwirkung zwischen Reagenz und Bindungspartner haben. Man kennt zahlreiche Markierungen die in ELISA-Tests oder in anderen immunologischen Tests verwendet werden können, wie z.B. Meerrettichperoxidase, Radioisotope, wie z.B. $^{14}$C und $^{131}$I, Eluorophore, wie z.B. seltene Erdmetalle oder Fluoreszin, Spin-labels usw.

In einer bevorzugten Ausführungsform der vorliegenden Erfindung wird ein ELISA (EIA) Test in einem Test-Kit, das nachfolgend beschrieben ist, unter Verwendung der erfindungsgemässen env/gag-Polypeptide, vorzugsweise unter Verwendung des erfindungsgemässen ENV(80)-GAG-15 Polypeptids, durchgeführt. In einer repräsentativen Ausführungsform dieses Tests werden drei verschiedene Proben getestet. Kugeln, die mit erfindungsgemässen env/gag-Polypeptiden beladen sind, werden mit Patientenproben (Serum), positiven und negativen Kontrollen bei 37°C während 1 Stunde inkubiert. Nach einem Waschvorgang zur Entfernung von nicht gebundenen Antikörpern werden die Kugeln mit anti-human-Ig-Meerrettichperoxidase (HRP)-Konjugat bei 37°C während einer Stunde inkubiert. Nach einem weiteren Waschvorgang zur Entfernung von nichtgebundenem HRP-Konjugat werden die Kugeln mit einer HRP-Substratlösung, o-Phenylendiamin (OPD), bei Raumtemperatur während 30 Minuten inkubiert. Die Enzym reaktion wird durch Zugabe von Schwefelsäure gestoppt und die gelbbraune Farbintensität, welche durch HRP entstanden ist, im Spektrophotometer bei 492 nm gemessen. Ausführliche Details dieses Testverfahrens sind in Beispiel 8 beschrieben.

Ein weiteres Verfahren zum Nachweis von AIDS-Antikörpern mittels der erfindungsgemässen env/gag-Polypeptide ist ein Enzymimmunoassay nach dem "Sandwich"-Prinzip. Dieses Testverfahren basiert auf den Arbeiten von R.I. Maiolini und ist in Immunological Methods 20, 25-34 (1978) beschrieben. Geeignete Trägermaterialien, die mit erfindungsgemässen env/gag-Polypeptiden beladen sind, werden mit Patientenproben und erfindungsgemässen env/gag-Polypeptiden, die mit Peroxidase konjugiert sind, inkubiert. Die immunologische Reaktion kann in einem oder in zwei Schritten durchgeführt werden. Bei der Durchführung in zwei Schritten erfolgt eine Waschung zwischen der ersten und zweiten Inkubation. Nach Beendigung der immunologischen Reaktion(en) erfolgt eine Waschung. Danach wird die Peroxidase-Aktivität, beispielsweise mit o-Phenylendiamin, gemessen.

Die env/gag-Polypeptide der vorliegenden Erfindung können jedoch nicht nur zum Nachweis von AIDS-Antikörpern, sondern auch zum Nachweis von AIDS-Viren oder Fragmenten davon verwendet werden, da diese Polypeptide zur Herstellung von AIDS-Antikörpern verwendet werden können.

Solche Antikörper können durch Injektion eines Impfstoffes, enthaltend ein env/gag-Polypeptid und ein physiologisch verträgliches Trägermaterial, in einen Säuger oder Vogel hergestellt werden. Die für die Injektion notwendige Menge Protein ist dem Fachmann bekannt oder kann nach bekannten Methoden bestimmt werden. Der im Zusammenhang mit der vorliegenden Erfindung verwendete Ausdruck "Trägermaterial" bezieht sich entweder auf bekannte Zusammensetzungen, die für die menschliche Verabreichung geeignet sind, oder auf bekannte, bei der Tierimpfung verwendete Adjuvantien.

Zur Tierimpfung eignen sich als Adjuvantien komplettes oder inkomplettes Freunds Adjuvans (nicht für den menschlichen Gebrauch geeignet), Adjuvans 65 (enthaltend Erdnussöl, Mannit-monoolein und Aluminiummonostearat), Mineralgele, z.B. Aluminiumhydroxyd, Aluminiumphosphat und Alaun, grenzflächenaktive Substanzen,. z.B. Hexadecylamin, Octadecylamin, Lyso-Lecithin, Dimethyldioctadecylammoniumbromid, Methoxyhexadecylglycerin und Pluronic Polyole, Polyanionen, z.B. Pyran, Dextransulfat, poly IC, Polyacrylsäure und Carbopol, Peptide, z.B. Dimethylglycin, sowie Oel-Emulsionen. Die env/gag-Polypeptide können auch nach Einbau in Liposomen oder andere Mikro-Trägermaterialien oder nach Kopplung an Polysaccharide, andere Proteine oder andere Polymere verabreicht werden.

In einem besonders typischen Beispiel folgen einige Wochen nach der ersten Impfung eine oder mehrere Zusatzimpfungen was einen hohen Gehalt an Antikörpern gegen das gag/env-Protein zur Folge hat. Diese können dann in an sich bekannter Weise isoliert werden.

Natürlich können auch monoklonale Antikörper nach der von Köhler und Milstein beschriebenen Methode hergestellt werden. Um verschiedene monoklonale Antikörper zu identifizieren, welche alle gegen das gleiche Antigen gerichtet sind, kann die von Stähli et al. beschriebene Methode [J. of Immunological Methods 32, 297-304 (1980)] verwendet werden.

Die nach den vorstehend beschriebenen Methoden erhältlichen anti-env/gag-Polypeptid-Antikörper können in verschiedenen diagnostischen Tests zur Erkennung von AIDS-Viren oder Fragmenten davon verwendet werden. Solche Tests können in Form von Radioimmunoassays, entweder in Lösung oder an festem Träger, durchgeführt werden. Es können aber auch Enzymimmunoassays durchgeführt werden. Diese Test können entweder direkt oder indirekt mittels eines zweiten Antikörpers der gegen die anti-env/gag-Polypeptid-Antikörper gerichtet ist, durchgeführt werden. Zahlreiche Enzymaktivitäten können an die Antikörper gekoppelt werden, z.B. Peroxidase, Glucoseoxidase, ß -Galactosidase und Alkalische Phosphatase.

Das Prinzip das vielen dieser Tests zugrunde liegt beruht darauf, dass man menschliches Serum oder andere biologische Flüssigkeiten, die im Verdacht stehen AIDS-Viren oder Fragmente davon zu enthalten, mit einer bekannten Menge von Antikörpern gegen ein env/gag-Polypeptid reagieren lässt, so dass Antigen/Antikörper-Komplexe entstehen können, und diese Komplexe in an sich bekannter Weise nachweist.

Der Fachmann wird auch erkennen, dass es viele andere Nachweisverfahren gibt bei denen anti-env/gag-Polypeptid Antiseren zum Einsatz kommen, wie z.B. verschiedene Agglutinationstests. Hierbei wird die Wechselwirkung zwischen Antikörpern und AIDS-Viren oder Fragmenten davon mittels Anordnungen, in denen Partikel mit anti-env/gag-Polypeptid-Antikörpern überzogen sind, nachgewiesen. Solche Partikel sind beispielsweise Latex-Kugeln, Liposomen, Erythrozyten, Polyacrylamidkugeln oder irgendein geeignetes Polymer.

Die vorstehend beschriebenen Verfahren zum Nachweis von AIDS-Viren oder Fragmenten davon, oder von Antikörpern gegen AIDS-Viren können in geeigneten Test-Kits bestehend aus einem Gefäss, welches ein env/gag-Polypeptid oder anti-env/gag-Polypeptid-Antikörper der vorliegenden Erfindung enthält, durchgeführt werden.

Das erfindungsgemässe Test-Kit zum Nachweis von AIDS-Antikörpern besteht aus:

(a) einem Gefäss mit Kugeln, die mit erfindungsgemässen Polypeptiden, vorzugsweise ENV(80)-GAG-15 Polypeptiden, beladen sind;

(b) einem Gefäss mit Enzym-Antikörper-Konjugat, vorzugsweise anti-human-Ig-HRP-Konjugat, zur Bestimmung der AIDS-Antikörper in menschlichen Serenproben;

(c) einem Gefäss mit menschlichem Serum, enthaltend eine bestimmte Menge von AIDS-Antikörpern, für die positive Kontrolle;

(d) einem Gefäss mit menschlichem Serum, welches frei von AIDS-Antikörpern ist, für die negative Kontrolle;

(e) einem Gefäss mit Enzym-Substrat-Tabletten, vorzugsweise o-Phenylendiamin Tabletten;

(f) einem Gefäss mit Testpuffer;

(g) einem Gefäss mit Enzym-Substrat-Gebrauchspuffer; und

(h) einem Gefäss mit Schwefelsäure, zur Beendigung der Enzymreaktion.

Obwohl nur die Verwendung von Kugeln im erfindungsgemässen AIDS-Test beschrieben ist, muss man wissen, dass jedes Trägermaterial, welches die Polypeptide der vorliegenden Erfindung binden kann, wie z.B. Mehrfach-Mikrotiterplatten oder Reagenzgläser, anstelle der Kugeln verwendet werden kann.

Eine ausführliche Beschreibung des erfindungsgemässen Test-Kits und seiner Verwendung zum Nachweis von AIDS-Antikörpern ist in Beispiel 8 offenbart.

Die folgenden, nicht einschränkenden Beispiele tragen zum besseren Verständnis der vorliegenden Erfindung bei. Diese Beispiele können besser verstanden werden, wenn sie im Zusammenhang mit den begleitenden Figuren gelesen werden. In diesen Figuren treten die folgenden Abkürzungen und Symbole auf: A,B,Bg,E,H,P,Pv,S,Sc,X und Xb bezeichnen Schnittstellen für die Restriktionsendonukleasen AatII, BamHI, BglI, EcoRI, HindIII, PstI, PvuII, SalI, ScaI, XhoI bzw. XbaI. repräsentiert die Promotoren der Gene bla, lacI und neo; repräsentiert die ribosomalen Bindungsstellen der Gene bla, cat, neo und lacI; repräsentiert die Terminatoren $t_o$und Tl; repräsentiert das regulierbare Promoter/Operator Element P $_{N25*/O}$; repräsentiert die ribosomalen Bindungsstellen RBSII und RBSII, 3A + 5A; → repräsentiert kodierende Regionen unter der Kontrolle dieser zuletzt genannten ribosomalen Bindungsstellen; repräsentiert die für die Replikation benötigte Region (repl.); repräsentiert kodierende Regionen für Dihydrofolatreduktase (dhfr), Chloramphenicolacetyltransferase (cat), lac Repressor (lacI), ß -Lactamase (bla) und Neomycinphosphotransferase (neo); repräsentiert das env(80)-Gen; und repräsentiert das gag Gen.

Figur 1 zeigt die synthetischen Oligonukleotidfragmente, aus welchen sich das synthetische env(80) Gen zusammensetzt, sowie die Nukleotidsequenz des env(80) Gens.

Figur 2 ist eine schematische Darstellung der Konstruktion des Plasmids pA-ENV-20. Im linken, oberen Teil dieser Figur ist der Zusammenbau der synthetischen Oligonukleotide zu 4 Untereinheiten und die Klonierung dieser Untereinheiten in pUC-Plasmide gezeigt. Der untere, linke und rechte Teil dieser Figur zeigt den Zusammenbau der 4 Untereinheiten, wodurch das Plasmid pA-ENV-20, enthaltend das env(80)-Gen als BamHI-Fragment, erhalten wird.

Figur 3 Teil (a) ist eine schematische Darstellung des Plasmids pDS5/RBSII,3A + 5A. Die Nukleotidsequenz des XhoI/XbaI-Fragments, welche für das regulierbare Promoter/Operator-Element $P_{N25*/O}$, die ribosomale Bindungsstelle RBSII,3A + 5A, das cat-Gen und den Terminator Tl kodiert, ist in Teil (b) gezeigt. Dort sind die Schnittstellen für die in Teil (a) gezeigten Restriktionsendonukleasen überstrichen während die für Chloramphenicolacetyltransferase mit 21 zusätzlichen aminoterminalen Aminosäuren kodierende Region, die unter der Kontrolle der ribosomalen Bindungsstelle RBSII,3A + 5A steht, unterstrichen ist. Zusätzlich wird der pBR322 Anteil des Plasmids pDS5/RBSII,3A + 5A schematisch gezeigt, wobei sich die gegebenen Positionsnummern auf die Nukleotidsequenz des Plasmids pBR322 [J.G. Sutcliffe, Cold Spring Harbor Symp. Quant. Biol, 43, pp. 77-90 (1979)] beziehen.

Figur 4 Teil (a) ist eine schematische Darstellung des Plasmids pDS8/RBSII. Die Nukleotidsequenz des XhoI/XbaI-Fragments, welche für das regulierbare Promoter/Operator-Element P $_{N25*/O}$, die ribosomale Bindungsstelle RBSII, das dhfr-Gen, den Terminator $t_o$, das cat-Gen und den Terminator Tl kodiert, ist in den Teilen (b) und (c) gezeigt. Dort sind die Schnittstellen für die in Teil (a) gezeigten Restriktionsendonukleasen überstrichen, während die für Dihydrofolatreduktase mit 6 zusätzlichen aminoterminalen Aminosäuren kodierende Region (DHFR), unterstrichen ist. Zusätzlich wird der pBR322 Anteil des Plasmids pDS8,RBSII schematisch gezeigt, wobei sich die gegebenen Positionsnummern auf die Nukleotidsequenz des Plasmids pBR322 [J.G. Sutcliffe, supra] beziehen.

Figur 5 Teil (a) ist eine schematische Darstellung des Plasmids pDMI,1. Die gesamte DNA-Sequenz dieses Plasmids ist in den Teilen (b), (c) und (d) gezeigt. Dort sind die Schnittstellen für die in Teil (a) gezeigten Restriktionsendonukleasen überstrichen während die für Neomycinphosphotransferase (neo) und den lac-Repressor (lacI) kodierenden Regionen unterstrichen sind.

Figur 6 ist eine schematische Darstellung der Konstruktion des Plasmids pENV(80)-DHFR.

Figur 7 ist eine schematische Darstellung der Konstruktion des Plasmids pENV(80)-GAG-15. In den Teilen (a), (b) und (c) wird die Isolierung der Fragmente 1, 2 bzw. 3 schematisch dargestellt. Zusätzlich wird in Teil (b) die Nukleotidsequenz des SstI/BglII Fragments des gag Gens gezeigt. In dieser Sequenz sind die Schnittstellen für die Restriktionsendonukleasen SstI, PvuII, HindIII und BGIII unterstrichen. Teil (d) zeigt den Zusammenbau der Fragmente 1, 2 und 3, wodurch das Plasmid pENV(80)-GAG-15 erhalten wird. Die für ENV(80)-GAG-15 kodierende Region ist durch einen Pfeil gekennzeichnet.

Figur 8 ist eine schematische Darstellung der Konstruktion des Plasmids pENV(80)-GAG-15( Δ cat). Die für ENV(80)-GAG-15 kodierende Region ist durch einen Pfeil gekennzeichnet.

Figur 9 offenbart die vollständige Nukleotidsequenz des ENV(80)-GAG-15 Gens und die davon abgeleitete Aminosäuresequenz des ENV(80)-GAG-15 Polypeptids.

Figur 10 zeigt ein typisches Elutionsprofil, welches mittels einer Sephacryl S-200 superfein Säule und ENV(80)-GAG-15 Polypeptid enthaltenden Bakterienextrakten erhalten wurde.

Figur 11 zeigt eine SDS-PAGE Analyse von ENV(80)-GAG-15 Sammelfraktionen, die nach wiederholter Chromatographie mittels Sephacryl S-200 superfein erhalten wurden. Spur 1: ENV(80)-GAG-15 Polypeptid enthaltende Bakterienextrakte, die auf die Säule aufgetragen wurden; Spuren 2-9: verschiedene ENV-(80)-GAG-15 Polypeptid Sammelfraktionen.

Die Konstruktion des Plasmids pENV(80)-GAG-15($\Delta$ cat), welches das erfindungsgemässe ENV(80)-GAG-15 Polypeptid exprimiert, die Herstellung des ENV(80)-GAG-15 Polypeptids in E. coli sowie seine Reinigung sind in den folgenden Beispielen im Detail beschrieben.

## Beispiel 1

### Synthese der synthetischen Oligonukleotidfragmente, welche das synthetische env(80)-Gen bilden.

Die synthetischen Oligonukleotidfragmente sind in Fig. 1 gezeigt. Sie wurden simultan an fester Phase, wie von Bannwarth und Jaiza in DNA 5, 413-419 (1986) beschrieben, hergestellt.

## Beispiel 2

### Konstruktion des Plasmids pA-ENV-20

#### A. Prinzipien

Die synthetischen Oligonukleotide wurden zu 4 Genblöcken mit verschiedenen Restriktionsschnittstellen an ihren Enden zusammengesetzt. Diese Genblöcke wurden dann einzeln in pUC-Plasmide subkloniert. Die resultierenden Subklone wurden danach mittels der im Plasmid pUC nur einmal vorkommenden Restriktionsschnittstelle AatII zusammengesetzt. Die Klonierungsstrategie wird im Detail in Figur 2 gezeigt.

#### B. Ligierung der synthetischen Oligonukleotide zu verschiedenen Genblöcken

Die lyophilisierten Oligonukleotide wurden in Wasser gelöst (DNA-Konzentration 10 nmol/ml) und während einer Stunde bei Raumtemperatur inkubiert. Jeweils 100 pmol der veschiedenen Oligonukleotide wurden mit 1 µl $\gamma$ [$^{32}$P]-ATP (2 pmol; 5000 Ci/mmol) und 0,5 Einheiten T4-Polynukleotidkinase (BRL, Basel) in 50 µl 50 mM Tris-HCl, pH 8,0, enthaltend 10 mM $MgCl_2$, während 5 Minuten bei 37°C behandelt. Zu den Mischungen, mit Ausnahme derjenigen, die die Endfragmente enthielten, wurden je 6 µl 0,5 mM ATP zugegeben. Danach wurden die Reaktionen durch Erhitzen der Mischungen während 5 Minuten bei 65°C beendet. Jeweils 10 µl der Mischungen wurden in 4 verschiedene Eppendorf-Hütchen gegeben, und zwar so, dass die gewünschten Genblöcke hergestellt werden konnten. Nach Zugabe von 5 µl IM Tris-HCl, pH 7.8, 1 µl 1 M $MgCl_2$, 2µl 5M NaCl und 32 µl Wasser wurden die Mischungen während 5 Minuten gekocht. Die Eppendorf-Hütchen wurden dann in kochendes Wasser gestellt. Danach wurden die Mischungen während 4 Stunden bei 4°C in einem Kühlraum langsam abgekühlt. Die Ligierung der Oligonukleotidefragmente wurde bei 12°C während 24 Stunden nach Zugabe von 10 µl 1M DTT (Dithiothreitol), 1 µl 100 mM ATP und 5 µl T4-DNA-Ligase (5 Weiss-Einheiten, Pharmacia, Uppsala) durchgeführt. Die erhaltenen Genblöcke wurden dann nach Zugabe von 12 µl 5M Lithiumacetat und 160 µl Isopropanol auf Trockeneis während 10 Minuten gefällt. Die Eppendorf-Hütchen wurden während 10 Minuten bei 12 000 Upm in einer Minizentrifuge zentrifugiert. Der Ueberstand wurde mit einer Pasteur-Pipette abgenommen und die DNA-Sedimente wurden mit 1 ml 80%igem Ethanol gewaschen. Danach wurden sie im Vakuum getrocknet, in 10µl Gelprobenpuffer (0,05% Bromphenolblau, 0,05% Xylencyanol, 10mM EDTA, pH 8,0) gelöst, und anschliessend auf einem 10%igen Polyacrylamidgel (50 × 50 × 1mm), enthaltend 1 × TBE [0,089M Tris-Borat, 0,089M Borsäure, 0,002M ETDA; T. Maniatis et al., Molecular Cloning, Cold Spring Harbor (1982)] während 20 Minuten bei 400 V aufgetrennt. Nach der Elektrophorese wurde das Gel während 5 Minuten in Ethidiumbromid (10 µg/ml) gefärbt. Die DNA-Banden wurden unter einer UV-Lampe bei einer Wellenlänge von 300 nm sichtbar gemacht. Die DNA-Banden der erwarteten Grössen wurden mittels eines Skalpells aus dem Gel herausgeschnitten. HaeIII vedaute ØX Phagen-DNA wurde als Marker verwendet. Die Gelstücke wurden in Vertiefungen (4 × 4mm) auf einem 0,7% Agarosegel überführt. Die Vertiefungen wurden anschliessend mit flüssiger 0,7% Agarose in 1 × TBE versiegelt, wodurch ein homogenes elektrisches Feld erreicht wurde. Vor jedes Gelstückchen wurde ein Stück NA45 Membran (Schleicher und Schuell, Dassel) gesetzt und die DNA wurde während 5 Minuten bei 300 V auf die Membranstückchen elektrotransferiert. Die Membranstückchen, enthaltend die elektrotransferierte DNA, wurden mit destilliertem Wasser gewaschen und in ein Eppendorf-Hütchen, enthaltend 250 µl 1,5 M Lithiumacetat, 50mM Tris-HCl, pH 8,0 und 10 mM EDTA, überführt. Die DNA wurde während 20 Minuten bei 65°C unter gelegentlichem Schütteln eluiert. Die Membranstückchen wurden aus den Eppendorf-Hütchen entfernt und die erhaltenen Mischungen wurden einmal mit 200 µl Phenol, das mit 1M Tris pH 8,0 gesättigt war, extrahiert. Danach wurden die Mischungen während 10 Minuten bei 12 000 Upm in einer Mikrozentrifuge zentrifugiert und die Ueberstände wurden verworfen. Die DNA wurde nach Zugabe von 20 µl 5 M Lithiumacetat und 440 µl Isopropanol während 10 Minuten auf Trockeneis gefällt. Danach wurde die DNA während 10 Minuten bei 12 000 Upm in einer Mikrozentrifuge nochmals sedimentiert. Die DNA-Sedimente wurden mit 80%igem Ethanol gewaschen, im Vakuum getrocknet, und in 10 µl Wasser gelöst.

C. Subklonierung der synthetischen Genfragmente in pUC-Vektoren

Die Plasmide pUC18 und pUC19 wurden von der Firma Pharmacia (Uppsala) erhalten. Ihre DNA-Konzentration betrug 0,4 µg/ml. 2 µl Plasmid DNA wurden mit 10 Einheiten der geeigneten Restriktionsendonukleasen in 1 × T4-Polymerasepuffer [T. Maniatis et al., supra] während 1 Stunde bei 37°C in einem Volumen von 50 µl verdaut. Die pUC18 Plasmid-DNA wurde mit BamHI und EcoRI zur Herstellung des Subklons 1 und mit BamHI und HindIII zur Herstellung des Subklons 4 verdaut. Die pUC19 Plasmid-DNA wurde mit EcoRI und PstI zur Herstellung des Subklons 3 und mit PstI und HindIII zur Herstellung des Subklons 3 verdaut (siehe Abbildung 2). Nach der Verdauung wurde die Plasmid-DNA, wie vorstehend beschrieben, einmal mit Phenol extrahiert, danach präzipitiert und getrocknet. Die DNA-Sedimente wurden in 10 µl Gelpuffer resuspendiert und auf einem 0,7%igen Agarosegel, enthaltend 1 × TBE und 1 µg/ml Ethidiumbromid, aufgetrennt. Die DNA-Banden wurden mittels UV-Licht bei einer Wellenlänge von 300 nm sichtbar gemacht. Ein Schlitz wurde dann vor jede DNA-Bande in das Gel geschnitten und ein Stück NA45-Membran wurde in diesen Schlitz gesteckt. Die DNA wurde auf die Membran während 10 Minuten bei 300 V elektrotransferiert. Die DNA wurde dann wie vorstehend beschrieben, eluiert und gereinigt. Das erhaltene DNA-Sediment wurde in 30 µl Wasser gelöst (Endkonzentration 0,1 µg/ml). Jeweils 1 µl der gereinigten und geschnittenen Plasmid-DNAs und 2 µl der verschiedenen Genblöcke wurden nach Zugabe von 1 µl 10-fach Ligasepuffer (0,5M Tris-HCl, pH 7,8, enthaltend 10mM $MgCl_2$, 100 mM DTT und 50mM Nacl) und 1 µl DNA-Ligase (1 Weiss-Einheit) in einem Volumen von 10 µl ligiert. Die Ligierungen wurden bei 20°C während 1 Stunde durchgeführt. Kontrolligierungen ohne Genblöcke wurden parallel dazu durchgeführt.

Der E. coli Stamm TB-1 wurde von BRL (Messing-Stammkit) erhalten. Zur Herstellung von kompetenten Zellen wurde eine einzelne Kolonnie des E.coli Stammes TB-1 in LB-Medium [10 g Bacto-tryptone, 5 g Bacto-yeast-Extrakt, 10 g NaCl pro Liter; T. Maniatis et al., supra] über Nacht bei 37°C in einem Schüttler wachsen gelassen. 500 µl dieser Uebernachtkultur wurden in 100 ml LB-Medium verdünnt und für weitere 2 Stunden bei 37°C wachsen gelassen. Die Zellen wurden mittels Zentrifugation während 10 Minuten bei 9000 Upm und 4°C gesammelt. Das Sediment wurde sorgfältig in 20 ml 50mM $CaCl_2$ resuspendiert und für 30 Minuten auf Eis gestellt. Danach wurden die Zellen erneut zentrifugiert und das Sediment wurde in 10 ml 50mM $CaCl_2$, enthaltend 20% Glycerin (V/V), gelöst. Die Zellen wurden in Portionen von 300 µl - schockgefroren und bei -80°C aufbewahrt, wodurch für mindestens 3 Monate die Transformationseffizienz erhalten blieb.

Zur Transformation wurden 10 µl 0,5M $MgCl_2$ und 0,1M $CaCl_2$, 10 µl 30% Polyethylenglykol, DNA und Wasser gemischt (Gesamtvolumen 100 µl). Nach Zugabe von 100 µl aufgetauten kompetenten Zellen wurde die Transformationsmischung während 20 Minuten auf Eis gestellt. Danach wurde sie während 10 Minuten bei Raumtemperatur inkubiert und nach Zugabe von 1 ml LB-Medium während 1 Stunde bei 37°C im Wasserbad inkubiert. Die Transformationsmischung wurde dann während 3 Minuten bei 12000 Upm in einer Minizentrifuge zentrifugiert. Der Ueberstand wurde verworfen, das Sediment wurde anschliessend in 100 µg LB-Medium resuspendiert und danach auf LB-Agarplatten, enthaltend 80 µg/ml Ampicillin, ausplattiert. Die Platten wurden über Nacht bei 37°C inkubiert.

Mit den Ligierungsansätzen der Subklone 1-4 wurden ungefähr 200 Transformanten pro Transformationsansatz erhalten. Die Kontrollansätze ergaben keine Transformanten. Einzelne Kolonien wurden mit einem Zahnstocher gepickt. Anschliessend wurden sie in ein Reagenzglas, enthaltend LB-Medium und 80 µg/ml Ampicillin, gegeben und während 6 Stunden bei 37°C unter kräftigem Schütteln wachsen gelassen. Die Zellen wurden während 10 Minuten bei 8000 Upm zentrifugiert. Danach wurden die Sedimente in 500 µl 50mM Tris-HCl, pH8.0, enthaltend 10mM EDTA, resuspendiert. Nach Zugabe von Lysozym (Endkonzentration: 1 mg/µl) wurden die Mischungen zuerst während 5 Minuten bei Raumtemperatur inkubiert und nach Zugabe von 25 µl 10% SDS (Natriumdodecylsulfat) und 50 µl 5M Kaliumacetat für 15 Minuten auf Eis gestellt. Die chromosomale DNA wurde dann während 15 Minuten in einer Minizentrifuge bei 12000 Upm sedimentiert. Die Ueberstände wurden in frische Reagenzgläser überführt und nach Zugabe von 1 µl RNase A (10 mg/ml) bei 37°C während 10 Minuten inkubiert. Die Plasmid-DNA wurde mittels Zugabe von Phenol, welches mit 1M Tris-HCl, pH 8,0 gesättigt war, präzipitiert. Die Phasen wurden mittels Zentrifugation bei 12000 Upm während 5 Minuten getrennt. Die Ueberstände wurden in frische Reagenzgläser überführt und ein gleiches Volumen an Chloroform wurde hinzugefügt. Die Phasen wurden erneut mittels Zentrifugation getrennt und die Ueberstände in neue Reagenzgläser überführt. Die Plasmid-DNA wurde dann während 10 Minuten bei Raumtemperatur durch Zugabe von 0,6 Volumen Isopropanol und 0,1 Volumen 5M Lithiumacetat gefällt. Die Mischungen wurden dann während 10 Minuten bei 12000 Upm in einer Minizentrifuge zentrifugiert. Die Sedimente wurden mit 80% Ethanol gewaschen und im Vakuum getrocknet. Die getrockneten Sedimente wurden in 50 µl Wasser gelöst. 10 µl davon wurde, wie vorstehend beschrieben, mit dem gewünschten Restriktionsenzym (10 Einheiten) verdaut, um die gewünschten Inserts freizusetzen (Gesamtvolumen 100 µl). Die DNA wurde dann präzipitiert, mit 80% Ethanol gewaschen und im Vakuum getrocknet. Die getrockneten Sedimente wurden in 10 µl Gelprobenpuffer gelöst und mittels eines 6%igen Polyacrylamidgels, wie vorstehend beschrieben, analysiert. Als Marker wurde DNA des Phagen ØX aufgetragen. Die Inserts aller vier Subklone zeigten die erwartete Grösse von 64, 55, 58 bzw. 69 Basenpaaren. Die Subklone erhielten die Bezeichnung pA1, pA2, pA3 und pA4.

D. Konstruktion des Plasmids pA2-3

0,2 µg pA2 bzw. pA3 Plasmid-DNA wurde mit 5 Einheiten der Restriktionsendonukleasen AatII und PstI in einem Gesamtvolumen von 100 µl während 1 Stunde bei 37°C verdaut. Die Mischungen wurden, wie vorstehend beschrieben, präzipitiert, mit 80% Ethanol gewaschen und getrocknet. Die DNA-Sedimente wurden in 10 µl Gelpuffer (wie oben beschrieben) gelöst und auf einem 0,8% Agarosegel enthaltend 1 x TBE und 1 µg/ml Ethidiumbromid, aufgetrennt. Das 500 Basenpaare enthaltende Fragment des Plasmids pA2 und das 2000 Basenpaare enthaltende Fragment des Plasmids pA3 wurden, wie vorstehend beschrieben, mittels eines NA45-Membranstückchens isoliert und gereinigt. Die erhaltenen DNA-Sedimente wurden in 5 µl Wasser gelöst. Die besagten Fragmente (je 2 µl) wurden mit 1 µl 10-fach Ligasepuffer (0,5M Tris-HCl, pH 7,8, 0,1M $MgCl_2$, 0,2M DTT, 10mM ATP), 1 µl T4-DNA-Ligase (1 Weiss-Einheit) und 4 µl Wasser versetzt. Die Ligierung wurde während 1 Stunde bei Raumtemperatur durchgeführt. Die ligierte DNA wurde dann in den Stamm TB-1, wie vorstehend beschrieben, transformiert. Die erhaltenen Transformanten wurden mittels des Minilysatverfahrens, das vorstehend beschrieben wurde, weiter analysiert. Das Insert der rekombinaten Plasmide wurde durch Spaltung mit den Restriktionsendonukleasen EcoRI und HindIII freigesetzt und zeigte nach der Auftrennung mittels 8%igem Polyacrylamidgel die erwartete Grösse von 114 Basenpaaren. Eines der erhaltenen rekombinanten Plasmide, welches die Genblöcke 2 und 3 des env-Genfragments enthielt, wurde pA2-3 genannt.

E. Konstruktion des Plasmids pA1-2-3

0,2 µg pA1 bzw. pA2-3 Plasmid-DNA wurden mit jeweils 5 Einheiten der Restriktionsendonukleasen AatII und EcoRI in einem Volumen von 100 µl während 1 Stunde bei 37°C verdaut. Die nachfolgende Bearbeitung der geschnittenen Plasmid-DNAs war die gleiche wie bei der Konstruktion des Plasmids pA2-3 (siehe auch Abbildung 2). Das Insert der nach Transformation erhaltenen rekombinanten Plasmide wurde mittels HindIII-Verdauung freigesetzt, da in der Polylinkerregion des Plasmids pUC18 eine zusätzliche HindIII-Schnittstelle vorhanden ist, und zeigte nach der Auftrennung mittels 8%igem Polyacrylamidgel die erwartete Grösse von ungefähr 200 Basenpaaren. Eines der erhaltenen Plasmide, welches die Genblöcke 1, 2 und 3 enthielt, wurde pA1-2-3 genannt.

F. Konstruktion und Sequenzanalyse des Plasmids pA-ENV-20

2 µg pA1-2-3 Plasmid-DNA wurden mit 20 Einheiten HindIII während 1 Stunde bei 37°C in einem Volumen von 300 µl verdaut. Die DNA wurde, wie vorstehend beschrieben, präzipitiert und auf einem 1%igen Agarosegel aufgetrennt. Das 200 Basenpaare enthaltende Fragment wurde, wie vorstehend beschrieben, mittels eines NA45-Membranstückchens isoliert und danach weitergereinigt. Das erhaltene DNA-Pellet wurde in 10 µl Wasser resuspendiert.

0.5 µg pA4 Plasmid-DNA wurde mit 10 Einheiten HindIII in einem Volumen von 100 µl während 1 Stunde bei 37°C verdaut. Die Enden wurden dann durch Zugabe von 10 Einheiten bakterieller Alkalischer Phosphatase, 40 µl 1M Tris-HCl, pH 8,9 und 160 µl Wasser während 2 Stunden bei 65°C dephosphoryliert.

Die Mischung wurde dreimal mit 400 µl Phenol, welches mit 1M Tris-HCl, pH 8,0, gesättigt war, extrahiert. Die Phasen wurden, wie vorstehend beschrieben, mittels Zentrifugation getrennt. Nach der dritten Phenol extraktion wurde die DNA nach Zugabe von 40 µl 5M Lithiumacetat und 800 µl Isopropanol während 10 Minuten auf Trockeneis präzipitiert. Danch wurde die Mischung während 10 Minuten bei 12000 Upm in einer Minizentrifuge zentrifugiert. Der Ueberstand wurde verworfen und das Sediment wurde dreimal mit 80% Ethanol gewaschen und danach während 15 Minuten in einer Vakuumzentrifuge getrocknet. Das getrocknet Sediment wurde in 5 µl Wasser gelöst.

2 µl HindIII verdaute und desphosphorylierte pA-4 Plasmid-DNA wurden mit 1 µl pA1-2-3 HindIII-Fragment nach Zugabe von 1 µl T4-DNA-Ligase (1 Weiss-Einheit), 1 µl 10-fach Ligasepuffer und 5 µl Wasser während 1 Stunde bei Raumtemperatur ligiert. Parallel dazu wurde eine Kontrollligierung ohne HindIII-Fragment des Plasmids pA1-2-3 durchgeführt. Mit diesen Ligierungsmischungen wurde dann der E.coli Stamm TB-1 transformiert. Die Mischung, welche das HindIII-Fragment enthielt, erbrachte ungefähr 500 rekombinante Kolonien während die Mischung ohne HindIII-Fragment 20 Kolonien zeigte . 12 rekombinante Plasmide wurden mittels des Minilysatverfahren, wie vorstehend beschrieben, isoliert. Nach dem Schneiden der DNA mit BamHI wurde ein erwartetes 246 Basenpaare enthaltendes Fragment aus 10 der 12 Plasmide freigesetzt. Probeverdauungen mit HindIII, PstI und EcoRI zeigten ferner das Vorhandensein der erwarteten Restriktionsschnittstellen HindIII, PstI und EcoRI. Eines der erhaltenen Plasmide, welches besagtes Fragment und besagte Restriktionsschnittstellen enthielt, wurde pA-ENV-20 genannt.

Mit dem Plasmid pA-ENV-20 transformierte E.coli TB-1 Zellen wurden bei der Deutschen Sammlung von Mikroorganismen (DSM) in Göttingen am 3. Oktober 1985 nach dem Budapester Vertrag hinterlegt [E.coli TB-1 (pA-ENV-20, DSM Nr.: 3516].

G. Sequenzierung des BamHI-Fragments des Plasmids pA-ENV-20

M13mp18RFI DNA wurde von der Firma Pharmacia (Uppsala) gekauft (0,1 µg/ml). 10 µl (1 µg) wurden mit 10 Einheiten BamHI während 1 Stunde bei 37°C verdaut. Die DNA wurde mit 10 Einheiten bakterieller Alkalischer Phosphatase während 2 Stunden bei 65°C in 40 µl 1M Tris-HCl, pH 8,0 und 240 µl Wasser desphoryliert. Die Mischung wurde dann, wie vorstehend beschrieben, mit Phenol extrahiert und präzipitiert. Das Sediment wurde in 20 µl Wasser resuspendiert.

2 µg pA-ENV-20 Plasmid-DNA wurden mit 20 Einheiten BamHI in einem Volumen von 200 µl verdaut. Das BamHI Fragment wurde, wie vorstehend beschrieben, aus einem 1% Agarosegel isoliert und in 10 µl Wasser gelöst.

1 µl BamHI geschnittene und desphosphorylierte M13mp18RFI DNA und 3 µl des pA-ENV-20 BamHI Fragments wurden nach Zugabe von 5 µl Wasser, 1 µl T4-DNA-Ligase (1 Weiss-Einheit) und 1 µl 10 × Ligasepuffer während 1 Stunde bei Raumtemperatur miteinander verknüpft. Kompetente E.coli JM105 Zellen (BRL, Basel, Stammkit) wurden dann mit dieser Mischung nach dem vorstehend beschriebenen Protokoll transformiert. Die 1-stündige Inkubation nach der Transformation wurde weggelassen und die Zellen wurden in Softagar, enthaltend Indikatorzellen, IPTG (Isopropyl-ß-D-thiogalactopyranosid) und X-Gal (5-Brom-4-chlor-3-indolyl-ß-D-galactosid) direkt auf LB-Platen ausplattiert, wie in dem M13-Handbuch, welches mit dem Sequenzierkit von BRL (Basel) mitgeliefert wurde, beschrieben ist. Eine Kontrolligierung ohne BamHI-Fragment wurde parallel dazu durchgeführt. Nach der Inkubation über Nacht bei 37°C zeigte die Kontrolligierung 20 blaue Plaques. Die Ligierung mit BamHI Fragment zeigte 23 blaue Plaques und zusätzlich ungefähr 180 weisse Plaques mit BamHI Fragment. Drei weisse Plaques wurden mit einem Zahnstocher gepickt und nach Ueberimpfung in 3 ml LB-Medium, enthaltend 10 µl Indikatorzellen (JM105, BRL, Basel, Stammkit) einer frischen Uebernachtkultur, unter kräftigem Schütteln während 5 Stunden bei 37°C wachsengelassen. Einzelsträngige DNA Matrizen wurden aus den Phagenüberständen, wie in dem BRL-M13 Handbuch beschrieben, hergestellt. Die Matrizen wurden dann mittels der Dideoxymethode von Sanger unter Verwendung eines 17-mer Primers (Pharmacia, Uppsala) sequenziert. Die Reaktionen sowie die Auftrennung der Nukleotide mittels Gelelektrophorese auf 0,4 mm starken Gelen, wurden, wie in dem BRL-M13 Handbuch beschrieben, durchgeführt. Nach der Trennung der Glasplatten wurde das Gel in 10% Methanol, 10% Ameisensäure und 80% Wasser während 15 Minuten fixiert. Das Gel wurde dann auf Whatman 3MM Papier gelegt und auf einem Geltrockner getrocknet. Das getrocknete Gel wurde über Nacht auf einen Kodak-XAR-Film gelegt. Alle sequenzierten Matrizen zeigten die richtige, vorhergesagte Sequenz des synthetischen env(80)-Gens.

Beispiel 3

Beschreibung der Plasmide, die für die Konstruktion der Plasmide pENV(80)-DHFR, pENV(80)-GAG-15 und pENV(80)-GAG-15( Δ cat) verwendet wurden

A. Prinzipien

Das Plasmid pDS8/RBSII (Fig. 4) wurde für die Konstruktion des Plasmids pENV(80)-DHFR ausgewählt. Dieses letztgenannte Plasmid wurde zusammen mit dem Plasmid pDS5/RBSII,3A+5A (Fig. 3) und einem geeigneten gag-Fragment für die Konstruktion des Plasmids pENV(80)-GAG-15 verwendet. Um die Expression des ENV(80)-GAG-15-Proteins zu verbessern wurde das Plasmid pENV(80)-GAG-15( Δ cat) konstruiert. Dies geschah mittels Deletion von Teilen des cat-Gens des Plasmids pENV(80)-GAG-15. Zur wirksamen Expression enthalten die vorstehend genannten Vektoren, das regulierbare Promoter/Operator-Element $P_{N25*/O}$ [D. Stüber et al., The EMBO J. 3, 3143-3148 (1984)] und die ribosomalen Bindungsstellen RBSII oder RBSII,3A+5A. Diese ribosomalen Bindungsstellen wurden mittels DNA-Synthese hergestellt und stimmen mit den ribosomalen Bindungsstellen, welche unter Kontrolle des E.coli Phagen T5 Promoters $P_{G25}$ [R. Gentz, Doktorarbeit, Universität Heidelberg, (1984)] stehen, überein. Aufgrund der hohen Effizienz dieser Expressionssignale können die vorstehend genannten Vektoren nur dann stabil in E.coli gehalten werden, wenn das Promoter/Operator-Element durch Bindung des lac-Repressors an die Operatoreinheit des Promoters $P_{N25*/O}$ reprimiert wird. Der lac-Repressor wird vom lacI-Gen kodiert. Da der Promotor $P_{N25*/O}$ nur dann effizient reprimiert wird, wenn genügende Mengen an Repressormolekülen vorhanden sind, wurde das lacI$^{q}$-Allel mit einem mutierten Promoter verwendet, wodurch eine verstärkte Transkription des lacI Gens stattfindet und somit auch eine ausreichende Synthese von Repressormolekülen. Das lacI$^{q}$-Allel ist Teil des Plasmids pDMI,1 (Fig. 5), welches mit den vorstehend genannten Plasmiden kompatibel ist und welches zusätzlich das neo-Gen trägt, welches den Zellen Kanamycin resistenz verleiht. E.coli Zellen, die mit den Plasmiden pDS5/RBSII,3A+5A, pDS8/RBSII oder Derivaten dieser Plasmide, enthaltend HTLV-III-Gene, transformiert werden, müssen deshalb auch das Plasmid pDMI,1 in sich tragen, um die erstgenannten Plasmide bei sich zu behalten. Die Induktion der Expression in diesen Systemen wird durch die Zugabe von IPTG zum Medium bei einer gewünschten Zelldichte bewirkt.

B. Plasmid pDS5/RBSII,3A+5A

Der Teil des Plasmids pDS5/RBSII,3A+5A (Fig. 3), der zwischen den Restriktionsschnittstellen für XbaI und XhoI liegt und die Replikationsregion sowie das Gen für β -Lactamase, das den Zellen Ampicillinresistenz verleiht, enthält, stammt aus dem Plasmid pBR322 (F. Bolivar et al., Gene, 2, pp. 95-113 [1977]; J. G. Sutcliffe, supra). Der verbleibende Teil des Plasmids trägt das regulierbare Promoter/Operator-Element $P_{N25*/O}$ (D. Stüber et al., supra) gefolgt von der ribosomalen Bindungsstelle RBSII,3A+5A, die Teil eines EcoRI/BamHI-Fragments ist. Es folgen Schnittstellen für die Restriktionsenzyme SalI, PstI und HindIII, das Promoter-freie Gene der Chloramphenicolacetyltransferase [R. Marcoli et al., FEBS Letters 110 , 11-14 (1980)] und der Terminator T1 des E.coli rrnB Operon [J. Brosius et al., J. Mol. Biol. 148, 107-127 (1981)].

C. Plasmid pDS8/RBSII

Das Plasmid pDS8/RBSII (Fig. 4) entspricht dem Plasmid pDS5/RBSII,3A+5A (Fig. 3) enthält jedoch zusätzlich zwischen dem regulierbaren Promoter/Operator-Element $P_{N25*/O}$ und dem cat-Gen die ribosomale Bindungsstelle RBSII gefolgt vom Gen der Dihydrofolatreduktase der Maus-Zellinie AT-3000 [Y.C.Y. Chang et al., Nature 275, 617-624 (1978); J.N. Masters and G. Attardi, Gene 21, 59-63 (1983)] und dem Terminator $t_o$ des E.coli-Phagen lambda [E. Schwarz et al., Nature 272, 410-414 (1978)].

D. Plasmid pDMI,1

Das Plasmid pDMI,1 (Fig. 5) trägt auf einem HindIII/SalI-Fragment das Gen der Neomycinphosphotransferase aus dem Transposon Tn5 [E. Beck et al., Gene 19, 327-336 (1982)], das E.coli Zellen Kanamycinresistenz verleiht, gefolgt vom lacI-Gen [P.J. Farabaugh, Nature 274, 765-769 (1978)] mit der Promotormutation $I^q$ [M.P. Calos, Nature, 274, pp. 762-765 (1978)], das den lac-Repressor kodiert. Ausserdem enthält das Plasmid pDMI,1 eine Region des Plasmids pACYC184 [A.C.Y. Chang and S.N. Cohen, J. Bacteriol. 134, 1141-1156 (1978)], die alle Informationen enthält, die für die Replikation und stabile Weitergabe an die Tochterzellen notwendig sind.

Beispiel 4

Konstruktion des Plasmids pENV(80)-DHFR

A. Prinzipien

Das Plasmid pENV(80)-DHFR wurde durch Einbau des BamHI-Fragments des Plasmids pA-ENV-20, welches das ENV(80)-Gen enthält, in die BamHI-Schnittstelle des Plasmids pDS8/RBSII (Fig. 6) konstruiert.

B. Isolierung des BamHI-Fragments des Plasmids pA-ENV-20, welches das ENV(80)-Gen enthält

1 pmol des BamHI-Fragments des Plasmids pA-ENV-20, welches das ENV(80)-Gen enthält, wurde wie vorstehend beschrieben, isoliert und 10 µl TE-Puffer (10mM Tris-HCl, pH 7,6 enthaltend 1mM EDTA) resuspendiert.

C. Herstellung linearisierter pDS8/RBSII Plasmid-DNA

3 pmol des Plasmids pDS8/RBSII wurden mit der Restriktionsendonuklease BamHI vollständig verdaut. Nach der Inaktivierung des Enzyms mittels Inkubation während 7 Minuten bei 65°C und Entfernung der Proteine mittels Phenolextraktion gefolgt von einer Etherbehandlung wurde die DNA mit Ethanol präzipitiert. Die DNA wurde in 41 µl 50mM Tris/HCl, pH 8.0, enthaltend eine Einheit Kalbsdarmphosphatase (CIP, Boehringer, Mannheim), resuspendiert und während 1 Stunde bei 37°C inkubiert. Nach der Hitzeinaktivierung des Enzmys (siehe oben) und der Entfernung der Proteine (siehe oben) wurde die DNA mit Ethanol präzipitiert. Nach der Resuspension der DNA wurde die linearisierte Plasmid DNA mittels Elektrophorese in einem 1% Agarosegel isoliert, anschliessend auf NA45 Membranfilter elektrotransferiert, nach der Elution Ethanol präzipitiert und letztlich in 100 µl TE-Puffer (siehe oben) resuspendiert. Ungefähr 1 pmol linearisierte, dephosphorylierte DNA des Plasmids pDS8/RBSII wurde erhalten.

D. Zusammenbau des Plasmids pENV(80)-DHFR

0,025 pmol isolierte DNA des Plasmids pDSB/RBSII wurden in 24 µl Ligasepuffer, enthaltend 1,8 Einheiten T4-DNA-Ligase (Boehringer, Mannheim), mit 0,05 pmol des isolierten env(80)-Gens während 6 Stunden bei 20°C inkubiert. Danach wurde das Enzym durch Inkubation während 7 Minuten bei 65°C inaktiviert.

E.coli M15 Zellen ($CaCl_2$ kompetent), welche das Plasmid pDMI,1 enthielten, wurden mit der ligierten DNA unter geeigneten Bedingungen transformiert. Nach der Selektion des Transformanten bei 37°C auf LB-Platten, enthaltend 100 µg/ml Ampicillin und 25 µg/ml Kanamycin, wurden Kulturen einzelner Transformanten in LB-Medium, enthaltend 100 µg/ml Ampicillin und 25 µg/ml Kanamycin, wachsen gelassen. Die Plasmid-DNA dieser Kulturen wurde mittels Standardmethoden isoliert und mittels Restriktionsanalyse unter Verwendung der Restriktionsendonucleasen XhoI und HindIII bezüglich Vorhandensein des env(80)-Gens und seiner Orientierung analysiert. Ein Plasmid, welches das en(80)-Gen in der richtigen Orientierung enthielt, wurde pENV(80)-DHFR genannt (Fig. 6).

Beispiel 5

Konstruktion des Plasmids pENV(80)-GAG-15 A. Prinzipien

Das Plasmid pENV(80)-GAG-15 wurde unter Verwendung der folgenden drei DNA-Fragmente konstruiert (Fig. 7): 1) das XhoI-BamHI Fragment des Plasmids pENV(80)-DHFR, enthaltend das regulierbare Promoter/Operator-Element $P_{N25*/0}$,RBSII und das env(80)-Gen (Fragment 1), 2) ein BamHI-Fragment, enthaltend Teile der gag-Region des HTLV-III-Virus (Fragment 2), und 3) das BamHI-XhoI-Fragment des Plasmids pDS5/RBSII,3A+5A, enthaltend eine Polylinkerregion, das cat-Gen, den Terminator T1, die Replikationsregion, und das bla-Gen (Fragment 3).

B. Isolierung des Fragments 1 aus dem Plasmid pENV(80)-DHFR

2 pmol des Plasmids pENV(80)-DHFR wurden mit einer limitierten Menge an Restriktionsendonuklease BamHI verdaut, sodass ungefähr 50% der DNA-Moleküle linear vorlagen. Nach der Inaktivierung des Enzyms mittels Inkubation während 7 Minuten bei 65°C und der Entfernung der Proteine mittels Phenolextraktion, gefolgt von einen Etherbehandlung, wurde die DNA mit Ethanol präzipitiert. Die DNA wurde in 50 µl 50 mM Tris/HCl, pH 8.0, enthaltend 2,5 Einheiten CIP, resuspendiert und danach während 1 Stunde bei 37°C inkubiert. Nach der Hitzeinaktivierung des Enzyms und der Entfernung der Proteine wurde die DNA mit Ethanol präzipitiert. Die DNA wurde resuspendiert und mit der Restriktionsendonuklease XhoI vollständig verdaut. Nach der Hitzeinaktivierung und Entfernung des Enzyms wurde die DNA mit Ethanol präzipitiert. Nach Resuspension der DNA wurde das Fragment 1 (siehe Fig. 7a) mittels Elektrophorese in einem 1%igen Agarosegel isoliert, danach auf NA45-Membranfilter elektrotransferiert, eluiert, mit Ethanol präzipitiert und in TE-Puffer resuspendiert. Ungefähr 0,15 pmol Fragment 1 in einem Gesamtvolumen von 120 µl wurden erhalten.

C. Isolierung des Fragments 2

1 pmol des Plasmids pÜ-GAG, welches das Plasmid pUC19 ist, das zusätzlich ein SstI-BglII Fragment der gag-Region eines HIV-Virus mit der in Fig. 7b gezeigten Sequenz enthält, wurde mit 12 Einheiten der Restriktionsendonuklease HindIII im geeigneten Puffer (T. Maniatis et al., supra) während 1 Stunde bei 37°C verdaut, für 10 Minuten auf 65°C erhitzt, danach auf 14°C abgekühlt und während 45 Minuten mit einer Einheit des Klenow-Fragments der E.coli DNA-Polymerase (Boehringer) in 50mM Tris-HCl, pH 7.6, enthaltend 10mM $MgCl_2$ , 1mM DTT und je 2 nmol der vier verschiedenen dNTPs, behandelt. Danach wurde die Reaktionsmischung mit Phenol/Chloroform (1:1) extrahiert und anschliessend nochmals mit Chloroform. Die DNA wurde in 0,3M Natriumacetat, pH 7,2, resuspendiert und anschliessend mit 2 Volumen Ethanol bei -20°C über Nacht präzipitiert. Das resultierende DNA-Pellet wurde in 10 µl 10mM Tris-HCl, pH 7.6, enthaltend 10mM $MgCl_2$ , 50 mM NaCl, aufgenommen und anschliessend mit 15 Einheiten der Restriktionsendonuklease PvuII während 1 Stunde bei 37°C verdaut. 5 µl der resultierenden Reaktionsmischung wurden zu 45 µl 50 mM Tris-HCl, pH 7.6, enthaltend 10mM $MgCl_2$ , 10mM DTT, 0,5mM ATP, 20 pmol 5'-phosphorylierter 8-mer BamHI-Linker mit der Sequenz 5'-CGGATCCG-3' (PL-Biochemicals) und eine Einheit $T_4$ -DNA-Ligase (Pharmacia), hinzugefügt und über Nacht bei 14°C inkubiert. Die Ligationsmischung wurde dann während 10 Minuten auf 65°C erhitzt und auf ein Volumen von 100 µl mit dem geeigneten Restriktionsverdauungspuffer verdünnt. Danach wurden 100 Einheiten der Restriktionsendonuklease BamHI zugegeben und es wurde während 2 Stunden bei 37°C inkubiert. Nach Phenol/Chloroform-Extraktion wurde die DNA mit Ethanol präzipitiert und anschliessend auf einem 2,5%igen Agarosegel (NuSieve-Agarose, FMC) in TAE-Puffer (40mM Tris-Acetat, enthaltend 1mM EDTA) aufgetrennt. Das 580 Basenpaare enthaltende BamHI-Restriktionsfragment (Fragment 2) (siehe Fig. 7b) wurde aus dem Gel herausgeschnitten, zweimal mit Phenol extrahiert, danach mit Chloroform extrahiert und mittels Ethanol präzipitiert. Die Nukleotidsequenz dieses DNA-Fragments ist in Fig. 9 (Nukleotide 253-835) gezeigt. Das Plasmid pUC19 kann von der Firma Pharmacia, Uppsala, bezogen werden.

D. Isolierung des Fragments 3 aus dem Plasmid pDS5/RBSII,3A+5A

3 pmol des Plasmids pDS5/RBSII,3A+5A wurden vollständig mit der Restriktionsendonuklease BamHI verdaut. Nach der Hitzeinaktivierung des Enzyms und der Entfernung der Proteine wurde die DNA mit Ethanol präzipitiert. Die DNA wurde in 50 µl 50 mM Tris/HCl, pH 8.0, enthaltend 2,5 Einheiten CIP resuspendiert und während 1 Stunde bei 37°C inkubiert. Nach der Hitzeinaktivierung des Enzyms und der Entfernung der Proteine wurde die DNA mit Ethanol präzipitiert. Die DNA wurde resuspendiert und vollständig mit der Restriktionsendonuklease XhoI verdaut. Nach Hitzeinaktivierung und Entfernung des Enzyms wurde die DNA mit Ethanol präzipitiert. Nach der Resuspension der DNA wurde das Fragment 3 (siehe Fig. 7c) mittels Elektrophorese auf einem 1%igen Agarosegel isoliert, anschliessend auf NA45-Membranfilter elektrotransferiert, danach eluiert, Ethanol präzipitiert und in TE-Puffer resuspendiert. Ungefähr 0,4 pmol des Fragments 3 wurden in einem Volumen von 40 µl erhalten.

E. Zusammenbau des Plasmids pENV(80)-GAG-15

0,01 pmol des Fragments 1, 0,02 pmol des Fragments 2 und 0,01 pmol des Fragments 3 wurden in 24 µl Ligasepuffer, enthaltend 0,5 Einheiten T4-DNA-Ligase (Boehringer, Mannheim), während 1 Stunde bei 15°C inkubiert. Danach wurde das Enzym durch Inkubation während 7 Minuten bei 65°C inaktiviert.

E.coli M15 Zellen ($CaCl_2$ kompetent), enthaltend das Plasmid pDMI,1 wurden, mit der ligierten DNA unter geeigneten Bedingungen transformiert. Nach der Selektion der Transformanten bei 37°C auf LB-Platten, enthaltend 100 µg/ml Ampicillin und 25 µg/ml Kanamycin, wurden einzelne Kolonnien in LB-Medium, enthaltend 100 µg/ml Ampicillin und 25 µg/ml Kanamycin, wachsen gelassen. Die DNA dieser Kulturen wurden mittels Standardmethoden isoliert und anschliessend auf 0,7% Agarosegelen analysiert. Die DNA mit der erwarteten Grösse wurde anschliessend mittels Restriktionsanalyse unter Verwendung der Restriktionsendonukleasen AluI, BamHI, HindIII, PstI und XhoI auf das Vorhandensein und die Orientierung der Gene ENV(80) und GAG analysiert. Ein Plasmid mit den gewünschten Restriktionsschnittstellen wurde pENV(80)-GAG-15 (siehe Fig. 7d) genannt.

Beispiel 6

Konstruktion des Plasmids pENV(80)-GAG-15( Δ cat)

A. Prinzipien

Das Plasmid pENV(80)-GAG-15( Δ cat) wurde durch Deletion bestimmter Teile des cat-Gens des Plasmids pENV(80)-GAG-15 konstruiert (siehe Fig. 8). Dies geschah durch den Zusammenbau der folgenden Fragmente des Plasmids pENV(80)-GAG-15 (siehe Abbildung 8): 1) das BglI-PvuII Fragment, enthaltend Teile des bla-Gens, den Promoter $P_{N25*/0}$, die ribosomale Bindungsstelle RBSII, das env(80)-Gen, die Polylinker-Region und denjenigen Teil des cat-Gens, welcher durch die Restriktionsschnittstelle PvuII begrenzt ist (Fragment 4) und 2) das SacI-BglI Fragment, enthaltend Teile des cat-Gens, den Terminator T1, die Replikationsregion und Teile des bla-Gens (Fragment 5).

B. Isolierung des Fragments 4 des Plasmids pENV(80)-GAG-15

4 pmol des Plasmids pENV(80)-GAG-15 wurden mit der Restriktionsendonuklease BglI vollständig verdaut. Nach der Hitzeinaktivierung des Enzyms und Entfernung der Proteine wurde die DNA mit Ethanol präzipitiert. Die DNA wurde resuspendiert und ein Drittel davon wurde mit der Restriktionsendonuklease PvuII vollständig verdaut. Nach der Hitzeinaktivierung und der Entfernung des Enzyms wurde die DNA mit Ethanol präzipitiert. Nach der Resuspension der DNA wurde das Fragment 4 mittels 1% Agarose-Gelelektrophorese isoliert, elektrophoretisch auf NA45-Membranfilter elektrotransferiert und nach der Elution mit Ethanol präzipitiert und in TE-Puffer, wie oben beschrieben, resuspendiert. Ungefähr 1 pmol Fragment 4 in einem Gesamtvolumen von 50 µl wurden erhalten.

C. Isolierung des Fragments 5 aus dem Plasmid pENV(80)-GAG-15

Ein Drittel der Probe, welche nach der Verdauung des Plasmids pENV(80)-GAG-15 mit der Restriktionsendonuklease BglI erhalten wurde (siehe Abschnitt B), wurde mit der Restriktionsendonuklease ScaI vollständig verdaut. Nach Hitzeinaktivierung und Entfernung der Proteine wurde die DNA mit Ethanol präzipitiert, danach resuspendiert. Das Fragment 5 wurde mittels 1% Agrose-Gelekektrophorese isoliert, auf NA45-Membranfilter elektrotransferiert, danach eluiert, Ethanol präzipitiert und in TE-Puffer resuspendiert. Ungefähr 1 pmol Fragment 5 in einem Gesamtvolumen von 50 µl wurde erhalten.

D. Zusammenbau des Plasmids pENV(80)-GAG-15( Δ cat)

0,002 pmol der Fragmente 4 und 5 wurden in 20 µl Ligasepuffer, enthaltend 2 Einheiten T4-DNA-Ligase (Boehringer, Mannheim) während 4 Stunden bei 22°C inkubiert. Danach wurde das Enzym mittels Inkubation bei 65°C während 7 Minuten inaktiviert.

E.coli M15 ($CaCl_2$ kompetent), enthaltend das Plasmid pDMI,1, wurden mit der ligierten DNA unter geeigneten Bedingungen transformiert. Nach der Selektion der Transformanten bei 37°C auf LB-Platten, enthaltend 100 µg/ml Ampicillin und 25 µg/ml Kanamycin, wurden verschiedene Kulturen in LB-Medium, enthaltend 100 µg/ml Ampicillin und 25 µg/ml Kanamycin, wachsen gelassen. Die DNA dieser Kulturen wurde mittels Standardmethoden isoliert und auf ihre Grösse mittels 0,7% Agarose-Gelelektrophorese getestet. Die DNA mit der erwarteten Grösse wurde anschliessend auf die Anwesenheit von Teilen des cat-Gens mittels Restriktionsanalyse unter Verwendung der Restriktionsendonukleasen HindIII, HpaII und XbaI analysiert. Ein Plasmid mit dem gewünschten Restriktionsschnittmuster wurde pENV(80)-GAG-15( Δ cat) genannt.

## Beispiel 7

### Herstellung und Reinigung des ENV(80)-GAG-15-Polypeptids

A. Prinzipien

Mittels des Plasmids pENV(80)-GAG-15( Δ cat) wurde das erfindungsgemässe ENV(80)-GAG-15-Polypeptid in E.coli hergestellt und nach Aufschluss der Zellen bis zur Homogenität gereinigt.

B. Herstellung des ENV(80)-GAG-15-Polypeptids in E.coli

E.coli W3110 Zellen (ATCC Nr. 27325), enthaltend Plasmid pDMI,1, wurden mit dem Plasmid pENV-(80)-GAG-15( Δ cat) transformiert und anschliessend bei 37°C in 2 x TY-Medium (16 g Bacotrypton, 10 g Hefeextrakt, 5 g NaCl pro Liter) enthaltend 100 µg/ml Ampicillin und 25 µg/ml Kanamycin, wachsen gelassen. Bei einer optischen Dichte von $OD_{600}$ =1,0 wurde IPTG zugegeben (Endkonzentration 2mM). Nach weiteren 6 Stunden Inkubation bei 37°C wurden die Zellen mittels Zentrifugation geerntet.

C. Aufbrechen der Zellen

Das Pellet wurde in 50 ml 10mM Tris-HCl, pH 8.0, enthaltend 2mM EDTA, 1mM DTT, resuspendiert. Die Zellen wurden mittels Zentrifugation (13000 Upm, 10 Minuten) geerntet und in 20 ml des gleichen Puffers resuspendiert. 10 mg Lysozym, welches in 1 ml $H_2O$ gelöst war, wurden hinzugefügt. Nach der Inkubation bei 37°C während 15 Minuten wurden 400 µl 1M $MgCl_2$ und eine Spatelspitze DNase I (Serva Nr. 18525) zur Mischung gegeben und es wurde für weitere 30 Minuten bei 37°C inkubiert.

Nach der Verdünnung der Mischung mit 10mM Tris-HCl, pH 8,0, enthaltend 1mM DTT, auf 60 ml Gesamtvolumen wurde diese, wie oben beschrieben, zentrifugiert und das Pellet wurde einmal mit 10mM Tris-HCl, pH 8,0, enthaltend 1mM DTT, gewaschen. Das Pellet wurde danach in 60 ml 10mM Tris-HCl, pH 8,0, enthaltend 150mM NaCl, 1mM DTT, 0,5% Triton X-114 (w/v), resuspendiert und während 15 Minuten bei 0°C, danach während 30 Minuten bei 37°C und erneut während 15 Minuten bei 0°C inkubiert. Das mittels Triton X-114 nichtgelöste Material wurde mittels Zentrifugation (13000 Upm, 10 Minuten) gesammelt und einmal mit 20 ml 10mM Tris-HCl, pH 8,0, enthaltend 1mM DTT, gewaschen. Das Pellet wurde anschliessend in 2 ml 10mM Tris-HCl, pH 8,0, enthaltend 1mM DTT, resuspendiert und danach in 10 ml 10mM Tris-HCl, pH 8,0, enthaltend 7M Guanidin-HCl und 1mM DTT, gelöst. Nichtgelöstes Material wurde mittels Zentrifugation entfernt und falls notwendig durch einen 0,45 μ Filter filtriert. Die geklärte Lösung wurde 1:15 mit 10mM Tris-HCl, pH 8,0, enthaltend 1mM DTT, verdünnt und bei Raumtemperatur für 10 Minuten gerührt. Das weisse Präzipitat wurde mittels Zentrifugation gesammelt und einmal mit 10mM Tris-HCl, pH 8,0, enthaltend 1mM DTT, gewaschen.

Das Pellet wurde in 5 ml 125mM Tris-HCl, pH 6,8, enthaltend 4% SDS, 5mM DTT, 0,02% $NaN_3$ gelöst, wodurch eine Proteinkonzentration von 20-50 $OD_{278nm}$ Einheiten/ml erhalten wurde, und anschliessen durch einen 0,45 μm Filter filtriert.

D. Säulenchromatographische Auftrennung der extrahierten ENV(80)-GAG-15-Polypeptide

Die Säulenchromatographie wurde mittels Sephacryl S-200 superfein (Pharmacia, Uppsala) unter Verwendung einer Glassäule (120 x 5 cm) durchgeführt.

2,3 l Sephacryl S-200 superfein wurden mit 125mM Tris-HCl, pH 6,8 (mittels Vakuumsterilfiltration entgast) gewaschen. Das Gel wurde dann in die Säule gemäss den Instruktionen des Herstellers überführt. Die Säule wurde anschliessend mit dem gleichen Puffer bei einer Durchflussrate von 300 ml/h gepackt. Nach Stabilisierung der Gelhöhe wurde das Pufferreservoir entfernt und ein Durchflussadapter installiert. Die Flussrichtung wurde umgekehrt und das Packen der Säule bei einer Durchflussrate von 300 ml/h wurde für einige Stunden fortgesetzt.

Das Gel wurde mit 10 l Laufpuffer (125 mM Tris-HCl, pH 6,8, enthaltend 0,1% SDS) bei einer Flussrate von 150 ml/h äquilibriert. Die Flussrate wurde danach auf 100 ml/h gesenkt. 100 $OD_{278}$ Einheiten des bakteriellen ENV(80)-GAG-15 Extrakts wurden für jeden Trennvorgang auf die Säule aufgetragen. Die Elution der Proteine wurde bei einer Wellenlänge von 278 nm verfolgt. Abbildung 10 zeigt ein typisches Eluationsprofil, welches mit ENV(80)-GAG-15-Polypeptid enthaltendem bakteriellen Extrakt und der vorstehend genannten Sephacryl S-200 superfein Säule erhalten wurde. Die das ENV(80)-GAG-15 enthaltenden Fraktionen wurden gesammelt (siehe Abbildung 10) und mittels SDS-PAGE (Polyacrylamidgelelektrophorese) analysiert (Fig. 11).

Beispiel 8

Enzymimmunoassay zum Nachweis von AIDS-Antikörpern in Patientenseren.

A. Herstellung von Kugeln, die mit ENV(80)-GAG-15 Polypeptid beladen sind

Das ENV(80)-GAG-15 Polypeptid aus Beispiel 7 wurde in ca. 15 l 0,05 Carbonat/Bicarbonatpuffer, pH 9,5, gelöst (Endkonz.: 1,4 $mOD_{278}$ /ml). 110'000 Polystyrolkugeln (Sperotech oder Precision Ball Plastics) wurden dann zu dieser Lösung gegeben. Nach einer Inkubation bei Raumtemperatur während 2 Stunden wurden die Kugeln mit ca. 20 l PBST-Lösung [Phosphat-gepufferte Salzlösung (PBS) enthaltend 0,05% Tween 20] gewaschen und anschliessend in ca. 15 l frischer PBST-Lösung bei Raumtemperatur während 30 Minuten inkubiert. Nach dem Absaugen der PBST-Lösung wurden die Kugeln in ca. 15 l PBS, enthaltend 0,05% Tween 20 (Merck), 1% Rinderserumalbumin (BSA), 0,1% Kathron (Christ) und 10% Saccharose (Fluka), bei 45°C während 12 bis 24 Stunden inkubiert, um unspezifische Bindungen zu reduzieren. Nach dem Absaugen der besagten Inkubationslösung wurden die Kugeln auf Whatman 3MM Filterpapier gegeben und bei 37°C während 1 Stunde getrocknet.

B. Herstellung von Kontrollseren

Das positive Kontrollserum (IgG-Fraktion in PBS) wurde, wie in dem Lehrbuch "Monoklonale Antikörper, Herstellung und Charakterisierung", Peters et al. Hrsg., Springer Verlag, Berlin, Seiten 192-196 (1985) beschrieben, aus menschlichem Serum, von dem man wusste, dass es AIDS-Antikörper enthielt, gewonnen.

Aus Sicherheitsgründen wurde das Serum vor der Weiterbehandlung, zur Inaktivierung aller infektiöser Agenzien, bei 56°C während 30 Minuten hitzebehandelt. Das negative Kontrollserum (hitzebehandelt bei 56°C während 2 Stunden und dann sterilfiltriert) wurde aus normalem Humanserum, welches keine AIDS-Antikörper enthielt und welches Hepatitis B Oberflächenantigen negativ war, gewonnen.

C. Materialien und Reagenzien des Test-Kits

Die folgenden Materialien und Reagenzien werden mit dem erfindungsgemässen Test-Kit mitgeliefert:

1. 100 Kugeln, beladen mit ENV(80)-GAG-15 Polypeptid.
2. 25 ml Anti-human-Ig-Peroxidaselösung (Peroxidase >2 U/ml, Tris 0,1 mol/l).
3. 0,3 ml normales Humanserum, welches keine AIDS-Antikörper enthält, als negative Kontrolle.
4. 0,3 ml Humanserum, welches AIDS-Antikörper enthält, als positive Kontrolle.
5. 10 o-Phenylendiamin Tabletten (200 µmol/Tablette, 24%).
6. 100 ml Testpuffer enthaltend PBS, 20% Newborn Kälberserum (bei 56°C während 30 Minuten hitzebehandelt und dann sterilfiltriert), 0,05% Tween 20 und 0,1% Kathon.
7. 50 ml Substratpuffer enthaltend 6 mmol/l Wasserstoffperoxid und 0,1 mol/l Citrat, pH 5,2 (25°C).
8. 110 ml Schwefelsäure (0,5 mol/l, 4,9%).
9. 125 Teströhrchen in 5 Ständern zu je 25 Röhrchen.
10. 1 Paar Pinzetten.

Die ungeöffneten Reagenzien sind bei 2-8°C bis zu dem auf der Packung angegebenen Verfalldatum stabil.

Die folgenden Hilfsmaterialien werden nicht mit dem erfindungsgemässen Test-Kit geliefert, sie werden jedoch zur Durchführung des erfindungsgemässen AIDS-Test benötigt.

1. Wasserbad (37°C), eventuell Inkubator (37°C).
2. Photometer mit einer Wellenlänge von 490 bis 492 nm und einem Absorptionsbereich von 0 bis 2,0 oder vorteilhaft bis 2.5. Die automatische Auswertung wird durch die Verwendung des ETA Photometer <Roche>, Artikel Nr. 10 2800 6, wesentlich vereinfacht.
3. Vorrichtung zum Waschen der Kugeln (z.B. Spritzflasche und Wasserstrahlpumpe). Besonders empfehlenswert ist die Verwendung des EIA Washer <Roche>, automatische Ausführung, Artikel Nr. 10 2803 0, oder manuelle Ausführung, Artikel Nr. 10 2804 9.
4. Mixer.
5. Präzisionspipetten: 0,01 ml, 0,25 ml, 1,0 ml, 5.0 ml (oder Dispenser von vergleichbarer Genauigkeit).
6. Folie, ParaFilm oder Stopfen zum Verschliessen bzw. Abdecken der Teströhrchen.

D. Vorgehen bei der Verwendung des Test-Kits

Beim Nachweis von AIDS-Antikörpern mittels des erfindungsgemässen Test-Kits wird vorzugsweise wie folgt vorgegangen:

(a) 0,01 ml des besagten positiven Kontrollserums in mindestens zwei Teströhrchen pipettieren;

(b) 0,01 ml des besagten negativen Kontrollserums in mindestens drei Teströhrchen pipettieren;

(c) 0,01 ml Patientenprobe in mindestens 2 Teströhrchen pipettieren;

(d) 1 ml des besagten Testpuffers und 1 Kugel, die mit ENV(80)-GAG-15 Polypeptid beladen ist, in jedes der vorstehend genannten Röhrchen geben;

(e) Teströhrchen schütteln oder mit Vortex mischen und 1 Stunde bei 37°C inkubieren;

(f) Flüssigkeit aus den inkubierten Teströhrchen absaugen (Wasserstrahlpumpe) und die Kugeln in den Teströhrchen 3× mit destilliertem oder entionisiertem Wasser (3-5 ml) waschen, zuletzt Wasser absaugen;

(g) 0,25 ml besagter Anti-human-Ig-Peroxidaselösung in jedes gewaschene Teströhrchen pipettieren;

(h) Teströhrchen schütteln oder mit Vortex mischen und ca. 1 Stunde bei 37°C inkubieren;

(i) Flüssigkeit aus den inkubierten Teströhrchen absaugen (Wasserstrahlpumpe) und die Kugeln in den Teströhrchen 3× mit destilliertem oder entionisiertem Wasser (3-5 ml) waschen, zuletzt Wasser absaugen;

(j) 1 Tablette o-Phenylendiamin in 5 ml besagtem Substratpuffer vollständig auflösen (Enzym-Substratlösung für 19 Teströhrchen ausreichend) und ein Teströhrchen für den Reagenzien-Leerwert (RL) vorbereiten (die Enzym-Substratlösung ist 1 Stunde bei Raumtemperatur (18-26°C) im Dunkeln haltbar);

(k) 0,25 ml der besagten Enzym-Substratlösung in jedes gewaschene Teströhrchen sowie in das vorbereitete Röhrchen für den Reagenzien-Leerwert pipettieren;

(l) Teströhrchen, inklusive RL, schütteln oder mit Vortex mischen und 30 Minuten bei Raumtemperatur (18-26°C) inkubieren;

(m) 1 ml Schwefelsäure in alle Röhrchen, inklusive RL, pipettieren, um die Reaktion zu stoppen;

(n) Teströhrchen schütteln oder mit Vortex mischen;

(o) Kontrollen und Patientenprobe(n) gegen den Reagenzienleerwert im Photometer bei 492 nm messen. Wird die Messung in den Teströhrchen vorgenommen, so sind dieselben aussen zu reinigen, bevor sie in das Photometer gestellt werden; und

(p) den Test wie folgt auswerten:

(i) Mittelwert der Absorptionen der Teströhrchen mit positivem Kontrollserum bestimmen;

(ii) Mittelwert der Absorptionen der Teströhrchen mit negativem Kontrollserum bestimmen;

(iii) Mittelwert der Absorptionen der Teströhrchen mit der (den) Patienten-Doppelprobe(n) bestimmen; und

(iv) die Mittelwerte der Patientenprobe(n) mit dem Cutoff-Wert von 0.25 $OD_{492}$ des erfindungsgemässen Test vergleichen.

Patientenproben mit einer gleich hohen oder höheren Absorption als der Cutoff-Wert sind als positiv zu betrachten.

Der Absorptionsmittelwert der negativen Kontrollen darf zwischen 0,020 und 0,15, der Absorptionsmittelwert der positiven Kontrollen zwischen 0,600 und 1,100 liegen. Liegt der Absorptionsmittelwert der negativen oder positiven Kontrolle ausserhalb des angegebenen Bereiches, muss ein technischer Fehler oder ein Zerfall der Reagenzien vermutet werden. In diesem Fall ist die Testreihe zu wiederholen.

E. Ergebnisse

Empflindlichkeit und Spezifität ENV(80)-GAG-15 EIA

Empfindlichkeit und Spezifität des wurden auf "Western blots" (WB) als Referenzmethode bezogen, wobei an eindeutig negativen Proben, mit Ausnahme jener aus einer bekannten Risikogruppe, keine WB-Analyse vorgenommen wurde. Dabei ergab sich: (a) eine Empfindlichkeit von 98,8% bei 173 WBbestätigten positiven Proben. In dieser Zahl sind 2 Seren enthalten, die auch in den von Abbott Laboratories und Organon vertriebenen Enzymimmunoassays, in denen ganze abgetötete viren als Antigen verwendet werden, nicht reaktiv waren; und (b) eine Spezifität von 99,8% bei 1674 negativen Proben.

Reaktivität bei randomisierten Blutspendern (Blutbank)

Die unter Routinebedingungen in der Blutbank durchgeführten Untersuchungen ergaben folgendes Bild:

| Anzahl der Blutspender | Nicht reaktiv | Anfänglich reaktiv | Wiederholt reaktiv | WB + | WB ±* (fraglich) | WB – |
|---|---|---|---|---|---|---|
| 1483 | 1481 | 2 | 2 | 0 | 1 | 1 |

* Im WB nur p24 nachweisbar.

Reaktivität bei bekannten Risikogruppen

In einem Vergleichsversuch wurden 364 Human-Seren im ENV(80)-GAG-15 EIA und im käuflichen Abbott anti HTLV-III/LAV EIA getestet. Diese Seren stammten von Drogenabhängigen, Homosexuellen, Hämophilen, Patienten mit AIDS-Symptomen, Patienten aus Tansania mit Lebererkrankungen und polytransfundierten Patienten. Die mit beiden Tests erhaltenen Ergebnisse wurden mittels WB-Analyse und/oder dem käuflichen Organon EIA überprüft. Ein Vergleich beider Tests ergab folgendes Bild:

| | | ENV(80)-GAG-15 EIA | | |
|---|---|---|---|---|
| | | + | +/- | - |
| | + | 158 | 0 | 6 |
| Abbott | +/- | 8 | 12 | 44 |
| EIA | - | 5 | 5 | 126 |

Wie zu sehen ist, erkannten beide Tests 158 positive Serumproben (157 Proben wurden als positiv bestätigt, eine Probe war WB-negativ) und 126 negative Serumproben (124 Proben wurden als negativ bestätigt, zwei Proben waren WB-positiv). 8 Serumproben, die im ENV(80)-GAG-15 EIA als positiv identifiziert wurden, waren im Abbott EIA fraglich (+/-) (7 Proben wurden als positiv bestätigt, eine Probe war WB-negativ). 5 Serumproben, die im ENV(80)-GAG-15 EIA als positiv identifiziert wurden, waren im Abbott EIA negativ (3 Proben wurden als positiv bestätigt, 2 Proben waren fraglich). Beide Tests erkannten 12 Proben, die fraglich waren (9 Proben wurden als negativ, 3 Proben als positiv bestätigt). 5 Serumproben, die im ENV(80)-GAG-15 EIA als fraglich identifiziert wurden, waren im Abbott EIA negativ (alle Proben wurden als negativ bestätigt). 6 Serumproben, die im ENV(80)-GAG-15 EIA als negativ identifiziert wurden, waren im Abbott EIA positiv (alle Proben wurden als negativ bestätigt). 44 Serumproben, die im ENV(80)-GAG-15 EIA als negativ identifiziert wurden, waren im Abbott EIA fraglich (alle Proben wurden als negativ bestätigt).

Zusammengefasst lässt sich feststellen, dass der erfindungsgemässe ENV(80)-GAG 15 EIA im Vergleich zum Abbott anti. HTLVIII/LAV EIA, eine viel klarere Antwort betreffend positive oder negative Ergebnisse gibt. Der Abbott EIA erkannte mindestens zwei positive Serumproben nicht, die im WB als positiv bestätigt wurden. 2 Serumproben waren sowohl im erfindungsgemässen ENV(80)-GAG 15 als auch in den käuflichen Abbott und Organon EIAs falsch-negativ.

## Ansprüche

1. Polypeptide mit einer Aminosäuresequenz der Formel

```
  1 M R G S E A Q Q H L L Q L T V W G I K Q L Q A R I L A V E R
 31 Y L K D Q Q L L G I W G C S G K L I C T T A V P W N A S W S
 61 N K L L E Q I W N N M T W M E W D R E I N N Y T G S A D T G
 91 H S S Q V S Q N Y P I V Q N I Q G Q M V H Q A I S P R T L N
121 A W V K V V E E K A F S P E V I P M F S A L S E G A T P Q D
151 L N T M L N T V G G H Q A A M Q M L K E T I N E E A A E W D
181 R V H P V H A G P I A P G Q M R E P R G S D I A G T T S T L
211 Q E Q I G W M T N N P P I P V G E I Y K R W I I L G L N K I
241 V R M Y S P T S I L D I R Q G P K E P F R D Y V D R F Y K T
271 L R A E Q A R I R R P A A K L G E I F R S (ENV(80)-GAG-15)
```

I

oder Fragmente davon oder Analoga, die sich von besagten Polypeptiden und besagten Fragmenten durch Aminosäuresubstitution(en) unterscheiden, die mindestens eine antigene und/oder immunogene determinante Gruppe des HTLV-III env-Proteins und des HTLV-III gag-Proteins aufweisen.

2. Ein Polypeptid gemäss Anspruch 1 mit der Aminosäuresequenz der Formel I.

3. Ein Polypeptid gemäss Ansppruch 1, welches ein Fragment des Polypeptids der Formel I ist.

4. Ein Polypeptid gemäss Anspruch 1, welches sich von den Polypeptiden gemäss den Ansprüchen 2 und 3 durch Aminosäuresubsitution(en) unterscheidet.

5. Ein Polypeptid gemäss einem der Ansprüche 1-4 mit einem zusätzlichen Methioninrest am Aminoende.

6. Ein Polypeptid gemäss einem der Ansprüche 1-5, welches in Bakterien hergestellt wurde.

7. Ein Polypeptid gemäss einem der Ansprüche 1-6, welches in E. coli hergestellt wurde.

8. Ein Polypeptid gemäss einem der Ansprüche 1-7 in im wesentlichen homogener Form.

9. Ein Gen, welches ein Polypeptid gemäss einem der Ansprüche 1-8 kodiert.

10. Ein Expressionsvektor, worin ein Gen gemäss Anspruch 9 operativ an eine Expressionskontrollsequenz gebunden ist.

11. Ein Expressionsvektor gemäss Anspruch 10, der in einem gram-negativen und/oder gram-positiven Bakterium replizieren kann.

12. Ein Expressionsvektor gemäss Anspruch 11, der in E. coli replizieren kann.

13. Ein Expressionsvektor gemäss Anspruch 11 oder 12, der das Plasmid pENV(80)-GAG-15 oder das Plasmid pENV(80)-GAG-15 ( Δ cat) ist.

14. Mit einem Expressionsvektor gemäss den Ansprüchen 10-13 transformiertes Bakterium.

15. Mit einem Expressionsvektor gemäss den Ansprüchen 10-13 transformierter E. coli Stamm.

16. Mit einem Expressionsvektor gemäss den Ansprüchen 10-13 transformierter E. coli M15 Stamm.

17. Ein Polypeptid gemäss einem der Ansprüche 1-8 als wesentlicher Bestandteil eines Impfstoffes.

18. Ein Polypeptid gemäss einem der Ansprüche 1-8 als Antigen.

19. Verfahren zur Herstellung eines Polypeptids gemäss einem der Ansprüche 1-8, dadurch gekennzeichnet, dass man ein Bakterium mit einem Expressionsvektor gemäss einem der Ansprüche 10-13 transformiert, dann unter geeigneten Wachstumsbedingungen kultiviert und das besagte Polypeptid isoliert und, falls gewünscht, reinigt.

20. Verfahren zur Herstellung von AIDS-Antikörpern, dadurch gekennzeichnet, dass man einem Säuger oder Vogel eine ausreichende Menge eines Polypeptids gemäss einem der Ansprüche 1-8 injiziert und die gebildeten Antikörper aus dem Serum dieser Tiere isoliert.

21. Verfahren zum Nachweis von Antikörpern gegen AIDS, dadurch gekennzeichnet, dass man

(a) ein Polypeptid gemäss einem der Ansprüche 1-8 an festem Trägermaterial immobilisiert;

(b) dieses immobilisierte Polypeptid mit einer menschlichen Serumprobe in Kontakt bringt,

(c) nicht-gebundenes Material durch Waschung entfernt; und

(d) die gewaschenen immobilisierten Polypeptid/Antikörper-Komplexe durch Zugabe eines markierten Reagenz, welches selektiv menschliche Antikörper erkennt, wie z.B. markiertes Staphylococcus aureus Protein A oder anti-human Ig, nachweist.

22. Verfahren gemäss Anspruch 21, bei dem als festes Trägermaterial eine Kugel verwendet wird und das markierte Reagenz, welches selektiv menschliche Antikörper erkennt, anti-human-Ig-Peroxidase-Konjugat ist, das man mit o-Phenylendiamin reagieren lässt, wodurch eine Farbänderung in Gegenwart besagter Peroxidase eintritt.

23. Verfahren gemäss Anspruch 21 oder 22, bei dem das Polypeptid von Anspruch 2 verwendet wird.

24. Verfahren zum Nachweis von AIDS-Viren oder Fragmenten davon in menschlichen Seren oder anderen biologischen Flüssigkeiten, dadurch gekennzeichnet, dass man

(a) eine menschliche Serumprobe oder eine andere Körperflüssigkeitsprobe mit einer bekannten Menge von Antikörpern gegen ein Polypeptid gemäss einem der Ansprüche 1-8 reagieren lässt; und

(b) die in der Reaktionsmischung entstandenen Antigen/Antikörper-Komplexe nachweist.

25. Verfahren gemäss Anspruch 24, bei dem die Antikörper mit einem Enzym markiert sind und die in der Reaktionsmischung entstandenen Antigen/Antikörper-Komplexe mittels Enzymimmunoassay nachgewiesen werden.

26. Verfahren zur Herstellung eines Impfstoffes, dadurch gekennzeichnet, dass man ein Polypeptid gemäss einem der Ansprüche 1-8 mit einem physiologisch verträglichen Trägermaterial mischt.

27. Impfstoffe enthaltend ein Polypeptid gemäss einem der Ansprüche 1-8 und ein physiologisch verträgliches Trägermaterial.

28. Antikörper gegen ein Polypeptid gemäss einem der Ansprüche 1-8.

29. Antikörper gemäss Anspruch 28, welches monoklonale Antikörper sind.

30. Die Verwendung eines Polypeptids gemäss einem der Ansprüche 1-8 zur Herstellung eines Impfstoffes gegen AIDS.

31. Die Verwendung eines Polypeptids gemäss einem der Ansprüche 1-8 zur Herstellung von AIDS-Antikörpern.

32. Die Verwendung eines Polypeptids gemäss einem der Ansprüche 1-8 zum Nachweis von AIDS-Antikörpern in menschlichen Seren oder anderen biologischen Flüssigkeiten.

33. Die Verwendung eines Polypeptids gemäss einem der Ansprüche 1-8 zum Nachweis von AIDS-Viren oder Fragmenten davon in menschlichen Seren oder anderen biologischen Flüssigkeiten.

34. Test-Kit zur Bestimmung von AIDS-Viren oder AIDS-Virusfragmenten bestehend aus einem Gefäss, welches AIDS-Antikörper gegen ein Protein gemäss einem der Ansprüche 1-8 enthält.

35. Test-Kit zur Bestimmung von AIDS-Antikörpern bestehend aus:

(a) einem Gefäss mit Kugeln, die mit einem Polypeptid gemäss einem der Ansprüche 1-8 beladen sind;

(b) einem Gefäss mit Enzym-Antikörper-Konjugat;

(c) einem Gefäss mit menschlichem Serum, welches AIDS-Antikörper enthält;

(d) einem Gefäss mit menschlichem Serum, welches frei von AIDS-Antikörpern ist;

(e) einem Gefäss mit Enzym-Substrat-Tabletten;

(f) einem Gefäss mit Testpuffer;

(g) einem Gefäss mit Enzym-Substrat-Gebrauchspuffer; und

(h) einem Gefäss mit Schwefelsäure.

36. Test-Kit gemäss Anspruch 35, worin das Gefäss mit Enzym-Antikörper-Konjugat anti-human-Ig-Peroxidase-Konjugat und das Gefäss mit Enzym-Substrat-Tabletten o-Phenylendiamin Tabletten enthält.

37. Test-Kit gemäss Anspruch 35 oder 36, worin ein Gefäss mit Kugeln, die mit Polypeptid gemäss Anspruch 2 beladen sind, enthalten ist.

Patentansprüche für folgenden Vertragsstaat: ES

1. Verfahren zur Herstellung von Polypeptiden mit einer Aminosäuresequenz der Formel

```
  1 M R G S E A Q Q H L L Q L T V W G I K Q L Q A R I L A V E R
 31 Y L K D Q Q L L G I W G C S G K L I C T T A V P W N A S W S
 61 N K L L E Q 1 W N N M T W M E W D R E I N N Y T G S A D T G
 91 H S S Q V S Q N Y P I V Q N I Q G Q M V H Q A I S P R T L N
121 A W V K V V E E K A F S P E V I P M F S A L S E G A T P Q D
151 L N T M L N T V G G H Q A A M Q M L K E T I N E E A A E W D
181 R V H P V H A G P I A P G Q M R E P R G S D I A G T T S T L
211 Q E Q I G W M T N N P P I P V G E I Y K R W I I L G L N K I
241 V R M Y S P T S I L D I R Q G P K E P F R D Y V D R F Y K T
271 L R A E Q A R I R R P A A K L G E I F R S (ENV(80)-GAG-15)
```

I

oder Fragmente davon oder Analoga, die sich von besagten Polypeptiden und besagten Fragmenten durch Aminosäuresubstitution(en) unterscheiden, die mindestens eine antigene und/oder immunogene determinante Gruppe des HTLV-III env-Proteins und des HTLV-III gag-Proteins aufweisen, dadurch gekennzeichnet, dass man ein Bakterium mit einem Expressionsvektor, der die Expression besagter Polypeptide oder Fragmente davon oder Analoga bewirkt transformiert, dann unter geeigneten Wachstumsbedingungen kultiviert und das besagte Polypeptid isoliert und, falls gewünscht, reinigt.

2. Verfahren gemäss Anspruch 1, bei dem ein Expressionsvektor, der die Expression eines Polypeptids mit der Aminosäuresequenz der Formel I bewirkt, verwendet wird.

3. Verfahren gemäss Anspruch 1, bei dem ein Expressionsvektor, der die Expression eines Fragments des Polypeptids der Formel I bewirkt, verwendet wird.

4. Verfahren gemäss Anspruch 1, bei dem ein Expressionsvektor verwendet wird, der die Expression eines Polypeptids, welches sich von dem gemäss Anspruch 2 definierten Polypeptid durch Aminosäuresubstitution(en) unterscheidet, bewirkt.

5. Verfahren gemäss Anspruch 1, bei dem ein Expressionsvektor verwendet wird, der die Expression eines Fragments, welches sich von dem Fragment gemäss Anspruch 3 durch Aminosäuresubstitution(en) unterscheidet, bewirkt.

6. Verfahren gemäss einem der Ansprüche 1-5, worin das Bakterium eine E. coli Zelle ist.

7. Verfahren gemäss Anspruch 6, worin die E. coli Zelle eine E. coli M15 Zelle ist.

8. Verfahren gemäss Anspruch 6 oder 7, worin der Expressionsvektor das Plasmid pENV(80)-GAG-15 oder das Plasmid pENV(80)-GAG-15( $\Delta$ cat) ist.

9. Verfahren zur Herstellung von AIDS-Antikörpern, dadurch gekennzeichnet, dass man einem Säuger oder Vogel eine ausreichende Menge eines gemäss einem der Ansprüche 1-5 definierten Polypeptids injiziert und die gebildeten Antikörper aus dem Serum dieser Tiere isoliert.

10. Verfahren zum Nachweis von Antikörpern gegen AIDS, dadurch gekennzeichnet, dass man

(a) ein gemäss einem der Ansprüche 1-5 definierten Polypeptid an festem Trägermaterial immobilisiert;

(b) dieses immobilisierte Polypeptid mit einer menschlichen Serumprobe in Kontakt bringt,

(c) nicht-gebundenes Material durch Waschung entfernt; und

(d) die gewaschenen immobilisierten Polypeptid/Antikörper-Komplexe durch Zugabe eines markierten Reagenz, welches selektiv menschliche Antikörper erkennt, wie z.B. markiertes Staphylococcus aureus Protein A oder anti-human Ig, nachweist.

11. Verfahren gemäss Anspruch 10, bei dem als festes Trägermaterial eine Kugel verwendet wird und das markierte Reagenz, welches selektiv menschliche Antikörper erkennt, anti-human-Ig-Peroxidase-Konjugat ist, das man mit o-Phenylendiamin reagieren lässt, wodurch eine Farbänderung in Gegenwart besagter Peroxidase eintritt.

12. Verfahren gemäss Anspruch 10 oder 11, bei dem das gemäss Anspruch 2 definierte Polypeptid verwendet wird.

13. Verfahren zum Nachweis von AIDS-Viren oder Fragmenten davon in menschlichen Seren oder anderen biologischen Flüssigkeiten, dadurch gekennzeichnet, dass man

(a) eine menschliche Serumprobe oder eine andere Körperflüssigkeitsprobe mit einer bekannten Menge von Antikörpern gegen ein gemäss einem der Ansprüche 1-5 definiertes Polypeptid reagieren lässt; und

(b) die in der Reaktionsmischung entstandenen Antigen/Antikörper-Komplexe nachweist.

14. Verfahren gemäss Anspruch 13, bei dem die Antikörper mit einem Enzym markiert sind und die in der Reaktionsmischung entstandenen Antigen/Antikörper-Komplexe mittels Enzymimmunoassay nachgewiesen werden.

15. Verfahren zur Herstellung eines Impfstoffes, dadurch gekennzeichnet, dass man ein gemäss einem der Ansprüche 1-5 definiertes Polypeptid mit einem physiologisch verträglichen Trägermaterial mischt.

16. Die Verwendung eines gemäss einem der Ansprüche 1-5 definierten Polypeptids zur Herstellung eines Impfstoffes gegen AIDS.

17. Die Verwendung eines gemäss einem der Ansprüche 1-5 definierten Polypeptids zur Herstellung von AIDS-Antikörpern.

18. Die Verwendung eines gemäss einem der Ansprüche 1-5 definierten Polypeptids zum Nachweis von AIDS-Antikörpern in menschlichen Seren oder anderen biologischen Flüssigkeiten.

19. Die Verwendung eines gemäss einem der Ansprüche 1-5 definierten Polypeptids zum Nachweis von AIDS-Viren oder Fragmenten davon in menschlichen Seren oder anderen biologischen Flüssigkeiten.

20. Test-Kit zur Bestimmung von AIDS-Viren oder AIDS-Virusfragmenten bestehend aus einem Gefäss, welches AIDS-Antikörper gegen ein gemäss einem der Ansprüche 1-5 definiertes Polypeptid enthält.

21. Test-Kit zur Bestimmung von AIDS-Antikörpern bestehend aus:

(a) einem Gefäss mit Kugeln, die mit einem gemäss einem der Ansprüche 1-5 definierten Polypeptid beladen sind;

(b) einem Gefäss mit Enzym-Antikörper-Konjugat;

(c) einem Gefäss mit menschlichem Serum, welches AIDS-Antikörper enthält;

(d) einem Gefäss mit menschlichem Serum, welches frei von AIDS-Antikörpern ist;

(e) einem Gefäss mit Enzym-Substrat-Tabletten;

(f) einem Gefäss mit Testpuffer;

(g) einem Gefäss mit Enzym-Substrat-Gebrauchspuffer; und

(h) einem Gefäss mit Schwefelsäure.

22. Test-Kit gemäss Anspruch 21, worin das Gefäss mit Enzym-Antikörper-Konjugat anti-human-Ig-Peroxidase-Konjugat und das Gefäss mit Enzym-Substrat-Tabletten o-Phenylendiamin-Tabletten enthält.

23. Test-Kit gemäss Anspruch 21 oder 22, worin ein Gefäss mit Kugeln, die mit gemäss Anspruch 2 definierten Polypeptid beladen sind, enthalten ist.

3. Dez. 1987
RAN 4105/102

Figur 1

```
BamHI ------------------ 1 ------------------ EcoRI ------------------ 2 ------------------ PstI
GATCCGAAGCTCAACAGCATCTGCTGCAACTCACTGTTTGGGGTATCAAACAGCTCCAGGCTCGAATTCTGGCTGTTGAACGTTACCTGAAAGATCAACAGCTCCTGGGTATCTGGGGCTGCAGT
    GCTTCGAGTTGTCGTAGACGACGTTGAGTGACAAACCCCATAGTTTGTCGAGGTCCGAGCTTAAGACCGACAACTTGCAATGGACTTTCTAGTTGTCGAGGACCCATAGACCCCGACGTCA

------------------ 3 ------------------ HindIII ------------------ 4 ------------------ BamHI
GGTAAACTCATCTGCACTACTGCTGTTCCTTGGAATGCTTCTTGGTCTAATAAGCTTCTGGAACAGATCTGGAATAACATGACTTGGATGGAGTGGGACCGTGAAATCAACAATTACACTG
CCATTTGAGTAGACGTGATGACGACAAGGAACCTTACGAAGAACCAGATTATTCGAAGACCTTGTCTAGACCTTATTGTACTGAACCTACCTCACCCTGGCACTTTAGTTGTTAATGTGACCTAG
```

Fig. 2

Konstruktion des Plasmids pA-ENV-20

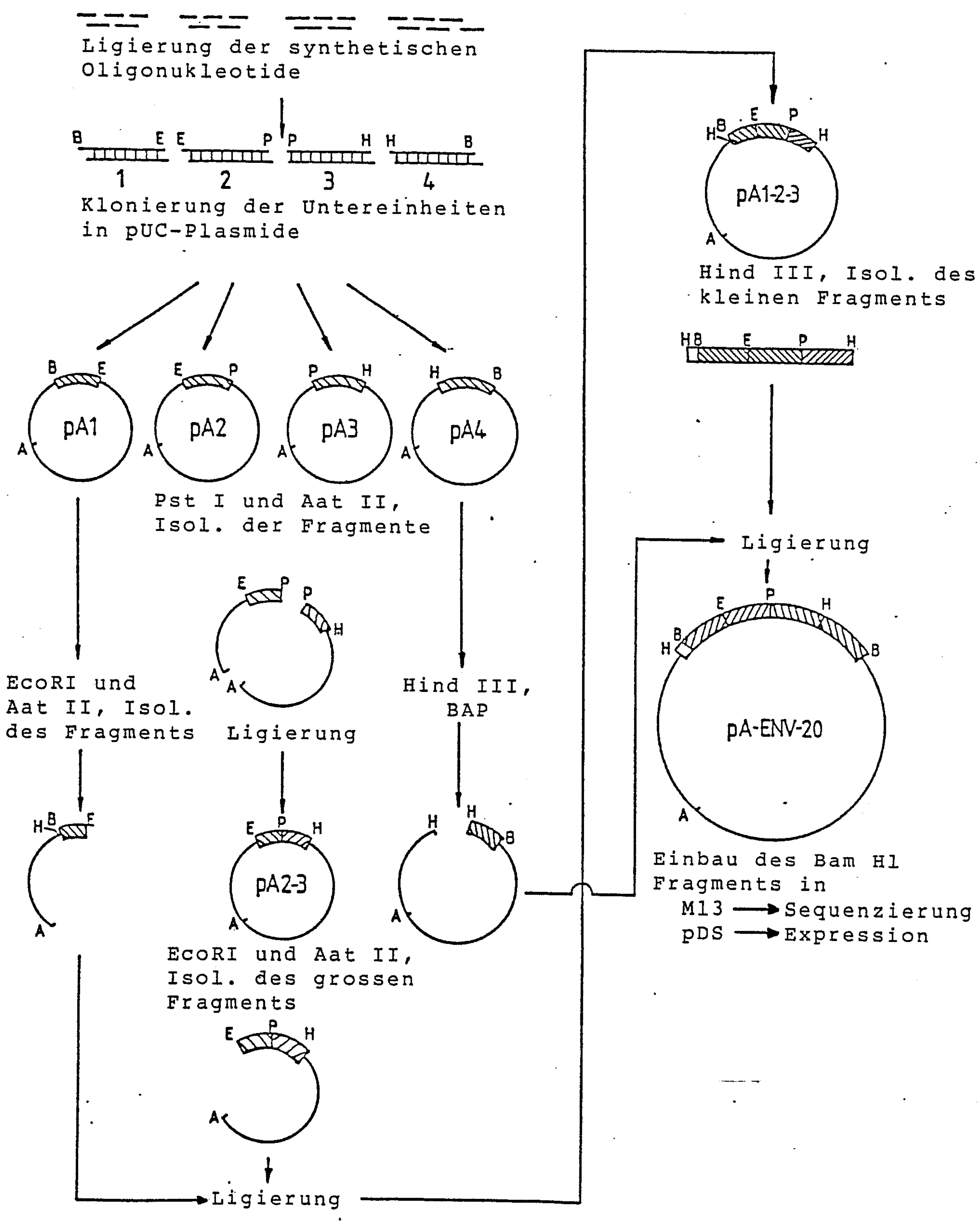

Fig. 3a

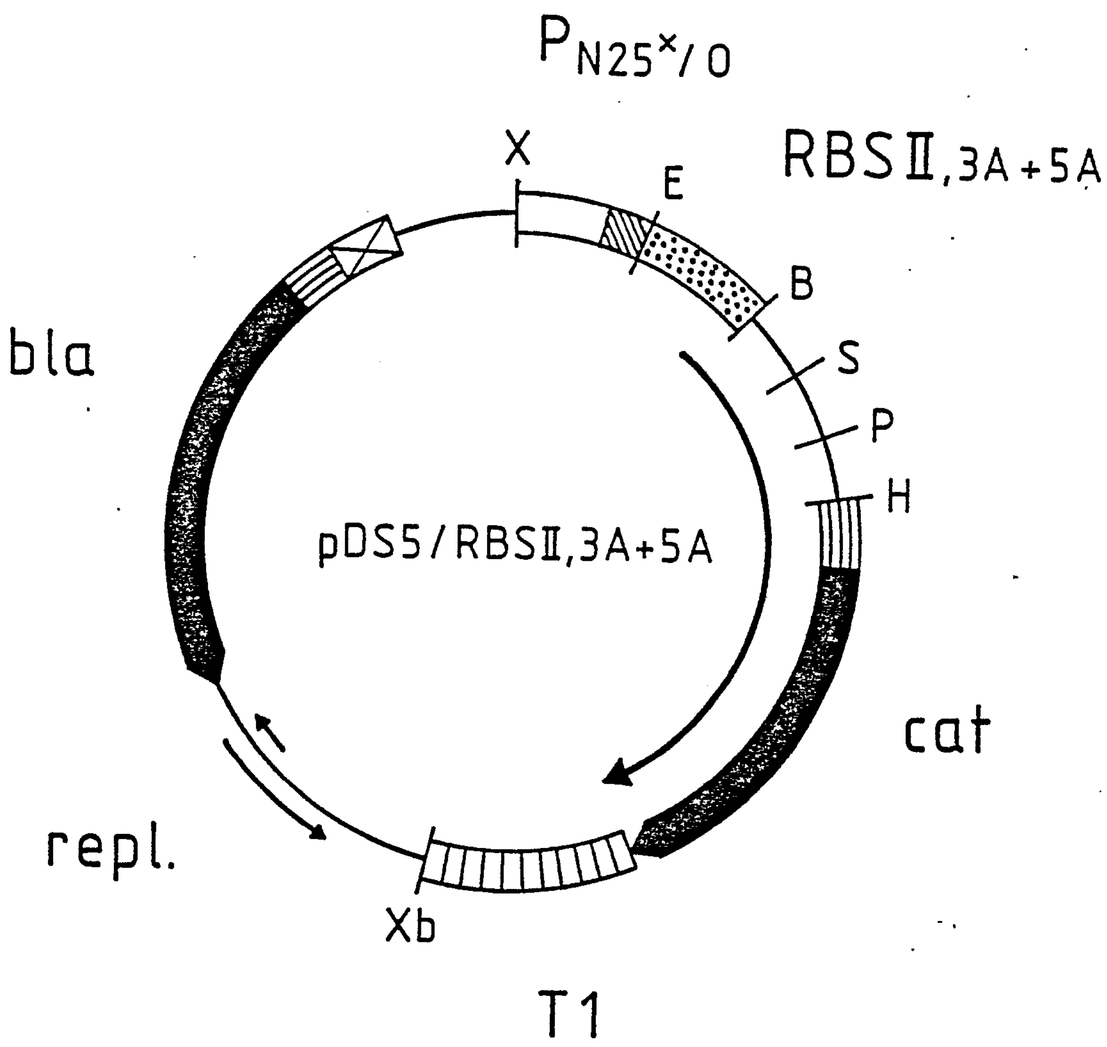

Fig. 3b

```
           10         20         30         40         50
   0 CCTCGAGGCT GGCATCCCTA ACATATCCGA ATGGTTACTT AAACAACGGA
  50 GGACTAGCGT ATCCCTTCGC ATAGGGTTTG AGTTAGATAA AGTATATGCT
 100 GAACTTTCTT CTTTGCTCAA AGAATCATAA AAAATTTATT TGCTTTCAGG
 150 AAAATTTTTC TGTATAATAG ATTCAAATTG TGAGCGGATA ACAATTTGAA
 200 TTCATTAAAG AGGAGAAATT AACTATGAGG GGATCCGTCG ACCTGCAGCC
 250 AAGCTTGGCG AGATTTTCAG GAGCTAAGGA AGCTAAAATG GAGAAAAAAA
 300 TCACTGGATA TACCACCGTT GATATATCCC AATGGCATCG TAAAGAACAT
 350 TTTGAGGCAT TTCAGTCAGT TGCTCAATGT ACCTATAACC AGACCGTTCA
 400 GCTGGATATT ACGGCCTTTT TAAAGACCGT AAAGAAAAAT AAGCACAAGT
 450 TTTATCCGGC CTTTATTCAC ATTCTTGCCC GCCTGATGAA TGCTCATCCG
 500 GAATTTCGTA TGGCAATGAA AGACGGTGAG CTGGTGATAT GGGATAGTGT
 550 TCACCCTTGT TACACCGTTT TCCATGAGCA AACTGAAACG TTTTCATCGC
 600 TCTGGAGTGA ATACCACGAC GATTTCCGGC AGTTTCTACA CATATATTCG
 650 CAAGATGTGG CGTGTTACGG TGAAAACCTG GCCTATTTCC CTAAAGGGTT
 700 TATTGAGAAT ATGTTTTTCG TCTCAGCCAA TCCCTGGGTG AGTTTCACCA
 750 GTTTTGATTT AAACGTGGCC AATATGGACA ACTTCTTCGC CCCCGTTTTC
 800 ACCATGGGCA AATATTATAC GCAAGGCGAC AAGGTGCTGA TGCCGCTGGC
 850 GATTCAGGTT CATCATGCCG TCTGTGATGG CTTCCATGTC GGCAGAATGC
 900 TTAATGAATT ACAACAGTAC TGCGATGAGT GGCAGGGCGG GGCGTAATTT
 950 TTTTAAGGCA GTTATTGGTG CCCTTAAACG CCTGGGGTAA TGACTCTCTA
1000 GCTTGAGGCA TCAAATAAAA CGAAAGGCTC AGTCGAAAGA CTGGGCCTTT
1050 CGTTTTATCT GTTGTTTGTC GGTGAACGCT CTCCTGAGTA GGACAAATCC
1100 GCCGCTCTAG AGC
```

2068

pBR322 A

4358

Fig. 4a

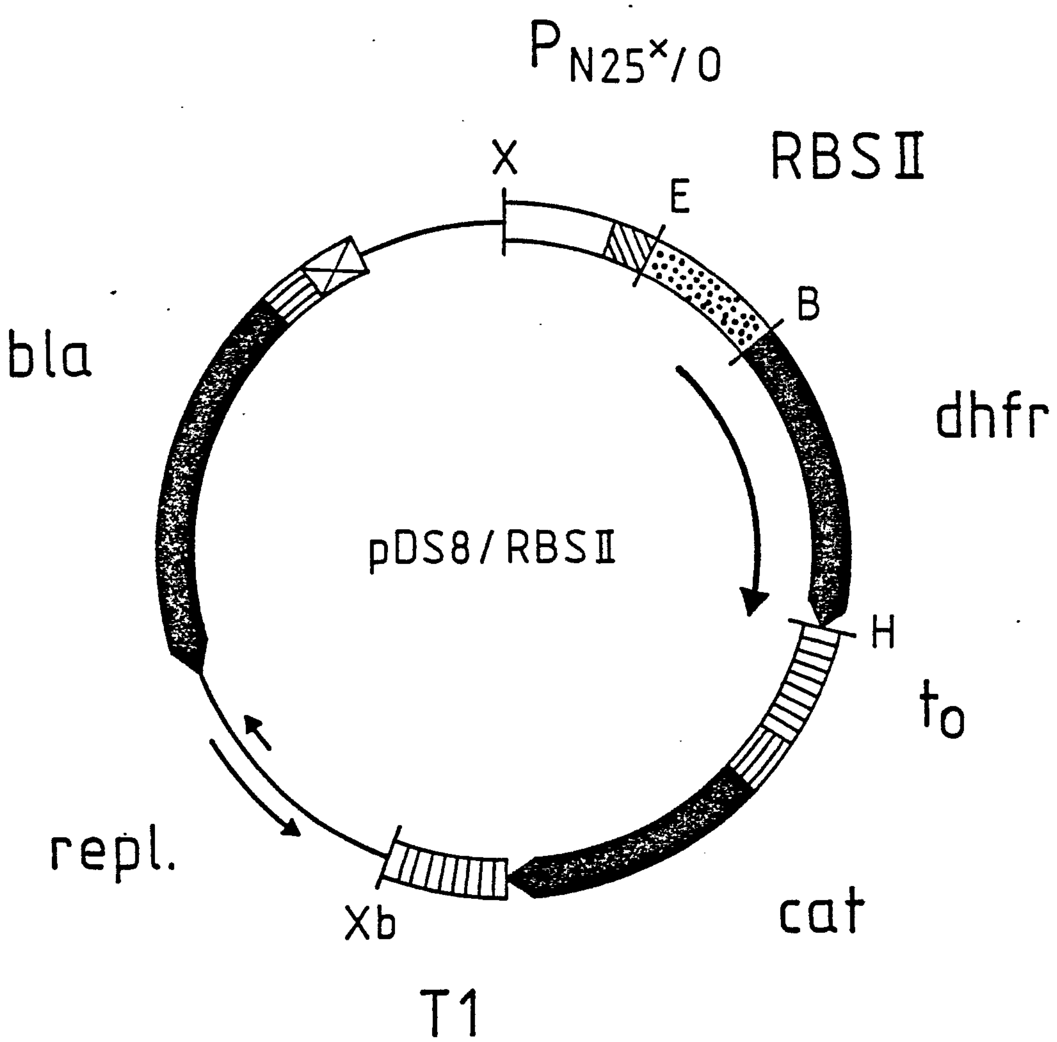

Fig. 4b

| | 10 | 20 | 30 | 40 | 50 |
|---|---|---|---|---|---|
| 0 | CCTCGAGGCT | GGCATCCCTA | ACATATCCGA | ATGGTTACTT | AAACAACGGA |
| 50 | GGACTAGCGT | ATCCCTTCGC | ATAGGGTTTG | AGTTAGATAA | AGTATATGCT |
| 100 | GAACTTTCTT | CTTTGCTCAA | AGAATCATAA | AAAATTTATT | TGCTTTCAGG |
| 150 | AAAATTTTTC | TGTATAATAG | ATTCAAATTG | TGAGCGGATA | ACAATTTGAA |
| 200 | TTCATTAAAG | AGGAGAAATT | AACTATGAGA | GGATCCGGCA | TCATGGTTCG |
| 250 | ACCATTGAAC | TGCATCGTCG | CCGTGTCCCA | AAATATGGGG | ATTGGCAAGA |
| 300 | ACGGAGACCT | ACCCTGGCCT | CCGCTCAGGA | ACGAGTTCAA | GTACTTCCAA |
| 350 | AGAATGACCA | CAACCTCTTC | AGTGGAAGGT | AAACAGAATC | TGGTGATTAT |
| 400 | GGGTAGGAAA | ACCTGGTTCT | CCATTCCTGA | GAAGAATCGA | CCTTTAAAGG |
| 450 | AGAGAATTAA | TATAGTTCTC | AGTAGAGAAC | TCAAAGAACC | ACCACGAGGA |
| 500 | GCTCATTTTC | TTGCCAAAAG | TTTGGATGAT | GCCTTAAGAC | TTATTGAACA |
| 550 | ACCGGAATTG | GCAAGTAAAG | TAGACATGGT | TTGGATAGTC | GGAGGCAGTT |
| 600 | CTGTTTACCA | GGAAGCCATG | AATCAACCAG | GCCACCTTAG | ACTCTTTGTG |
| 650 | ACAAGGATCA | TGCAGGAATT | TGAAAGTGAC | ACGTTTTTCC | CAGAAATTGA |
| 700 | TTTGGGGAAA | TATAAACTTC | TCCCAGAATA | CCCAGGCGTC | CTCTCTGAGG |
| 750 | TCCAGGAGGA | AAAAGGCATC | AAGTATAAGT | TTGAAGTCTA | CGAGAAGAAA |
| 800 | GACTAACAGG | AAGATGCTTT | CAAGTTCTCT | GCTCCCCTCC | TAAAGCTATG |
| 850 | CATTTTTATA | AGACCATGGG | ACTTTTGCTG | GCTTTAGATC | CGGCCAAGCT |
| 900 | TGGACTCCTG | TTGATAGATC | CAGTAATGAC | CTCAGAACTC | CATCTGGATT |
| 950 | TGTTCAGAAC | GCTCGGTTGC | CGCCGGGCGT | TTTTTATTGG | TGAGAATCCA |
| 1000 | AGCTAGCTTG | GCGAGATTTT | CAGGAGCTAA | GGAAGCTAAA | ATGGAGAAAA |
| 1050 | AAATCACTGG | ATATACCACC | GTTGATATAT | CCCAATGGCA | TCGTAAAGAA |
| 1100 | CATTTTGAGG | CATTTCAGTC | AGTTGCTCAA | TGTACCTATA | ACCAGACCGT |
| 1150 | TCAGCTGGAT | ATTACGGCCT | TTTTAAAGAC | CGTAAAGAAA | AATAAGCACA |

Fig. 4c

| | 10 | 20 | 30 | 40 | 50 |
|---|---|---|---|---|---|
| 1200 | AGTTTTATCC | GGCCTTTATT | CACATTCTTG | CCCGCCTGAT | GAATGCTCAT |
| 1250 | CCGGAATTTC | GTATGGCAAT | GAAAGACGGT | GAGCTGGTGA | TATGGGATAG |
| 1300 | TGTTCACCCT | TGTTACACCG | TTTTCCATGA | GCAAACTGAA | ACGTTTTCAT |
| 1350 | CGCTCTGGAG | TGAATACCAC | GACGATTTCC | GGCAGTTTCT | ACACATATAT |
| 1400 | TCGCAAGATG | TGGCGTGTTA | CGGTGAAAAC | CTGGCCTATT | TCCCTAAAGG |
| 1450 | GTTTATTGAG | AATATGTTTT | TCGTCTCAGC | CAATCCCTGG | GTGAGTTTCA |
| 1500 | CCAGTTTTGA | TTTAAACGTG | GCCAATATGG | ACAACTTCTT | CGCCCCCGTT |
| 1550 | TTCACCATGG | GCAAATATTA | TACGCAAGGC | GACAAGGTGC | TGATGCCGCT |
| 1600 | GGCGATTCAG | GTTCATCATG | CCGTCTGTGA | TGGCTTCCAT | GTCGGCAGAA |
| 1650 | TGCTTAATGA | ATTACAACAG | TACTGCGATG | AGTGGCAGGG | CGGGGCGTAA |
| 1700 | TTTTTTTAAG | GCAGTTATTG | GTGCCCTTAA | ACGCCTGGGG | TAATGACTCT |
| 1750 | CTAGCTTGAG | GCATCAAATA | AAACGAAAGG | CTCAGTCGAA | AGACTGGGCC |
| 1800 | TTTCGTTTTA | TCTGTTGTTT | GTCGGTGAAC | GCTCTCCTGA | GTAGGACAAA |
| 1850 | TCCGCCGCTC | TAGAGC | | | |

2068

pBR322 A

4358

Fig. 5a

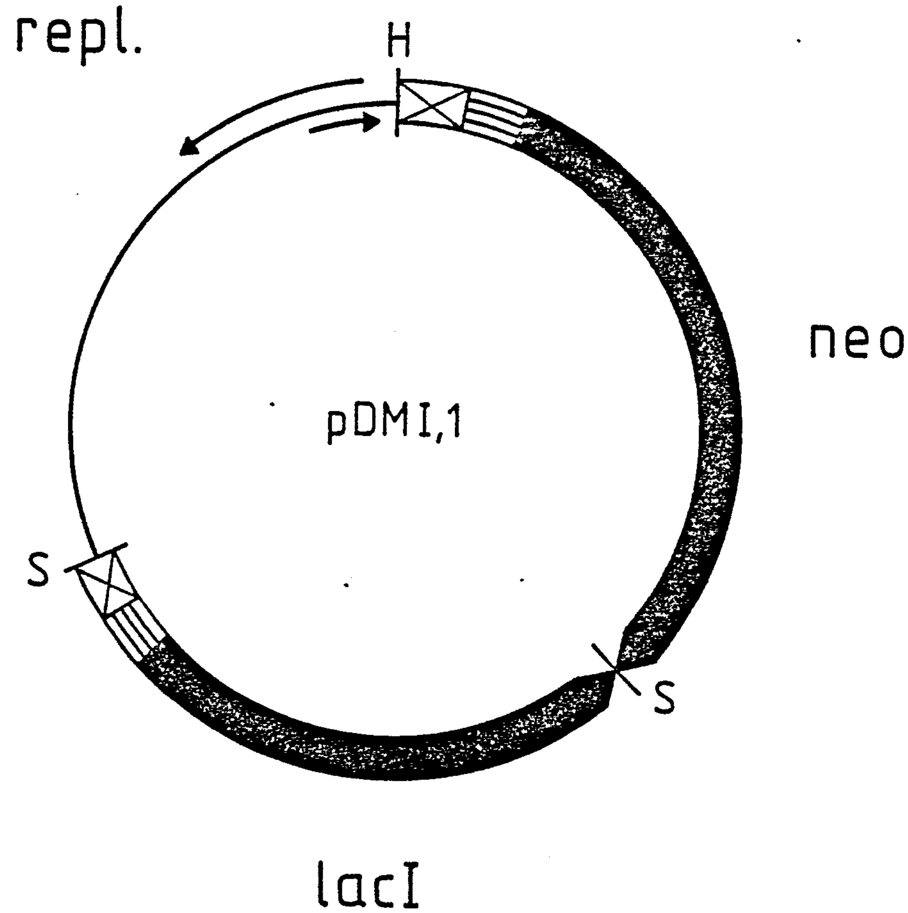

Fig. 5b

| | 10 | 20 | 30 | 40 | 50 |
|---|---|---|---|---|---|
| 0 | AAGCTTCACG | CTGCCGCAAG | CACTCAGGGC | GCAAGGGCTG | CTAAAGGAAG |
| 50 | CGGAACACGT | AGAAAGCCAG | TCCGCAGAAA | CGGTGCTGAC | CCCGGATGAA |
| 100 | TGTCAGCTAC | TGGGCTATCT | GGACAAGGGA | AAACGCAAGC | GCAAAGAGAA |
| 150 | AGCAGGTAGC | TTGCAGTGGG | CTTACATGGC | GATAGCTAGA | CTGGGCGGTT |
| 200 | TTATGGACAG | CAAGCGAACC | GGAATTGCCA | GCTGGGGCGC | CCTCTGGTAA |
| 250 | GGTTGGGAAG | CCCTGCAAAG | TAAACTGGAT | GGCTTTCTTG | CCGCCAAGGA |
| 300 | TCTGATGGCG | CAGGGGATCA | AGATCTGATC | AAGAGACAGG | ATGAGGATCG |
| 350 | TTTCGCATGA | TTGAACAAGA | TGGATTGCAC | GCAGGTTCTC | CGGCCGCTTG |
| 400 | GGTGGAGAGG | CTATTCGGCT | ATGACTGGGC | ACAACAGACA | ATCGGCTGCT |
| 450 | CTGATGCCGC | CGTGTTCCGG | CTGTCAGCGC | AGGGGCGCCC | GGTTCTTTTT |
| 500 | GTCAAGACCG | ACCTGTCCGG | TGCCCTGAAT | GAACTGCAGG | ACGAGGCAGC |
| 550 | GCGGCTATCG | TGGCTGGCCA | CGACGGGCGT | TCCTTGCGCA | GCTGTGCTCG |
| 600 | ACGTTGTCAC | TGAAGCGGGA | AGGGACTGGC | TGCTATTGGG | CGAAGTGCCG |
| 650 | GGGCAGGATC | TCCTGTCATC | TCACCTTGCT | CCTGCCGAGA | AAGTATCCAT |
| 700 | CATGGCTGAT | GCAATGCGGC | GGCTGCATAC | GCTTGATCCG | GCTACCTGCC |
| 750 | CATTCGACCA | CCAAGCGAAA | CATCGCATCG | AGCGAGCACG | TACTCGGATG |
| 800 | GAAGCCGGTC | TTGTCGATCA | GGATGATCTG | GACGAAGAGC | ATCAGGGGCT |
| 850 | CGCGCCAGCC | GAACTGTTCG | CCAGGCTCAA | GGCGCGCATG | CCCGACGGCG |
| 900 | AGGATCTCGT | CGTGACCCAT | GGCGATGCCT | GCTTGCCGAA | TATCATGGTG |
| 950 | GAAAATGGCC | GCTTTTCTGG | ATTCATCGAC | TGTGGCCGGC | TGGGTGTGGC |
| 1000 | GGACCGCTAT | CAGGACATAG | CGTTGGCTAC | CCGTGATATT | GCTGAAGAGC |
| 1050 | TTGGCGGCGA | ATGGGCTGAC | CGCTTCCTCG | TGCTTTACGG | TATCGCCGCT |
| 1100 | CCCGATTCGC | AGCGCATCGC | CTTCTATCGC | CTTCTTGACG | AGTTCTTCTG |
| 1150 | AGCGGGACTC | TGGGGTTCGA | AATGACCGAC | CAAGCGACGC | CCAACCTGCC |

Fig. 5c

| | 10 | 20 | 30 | 40 | 50 |
|---|---|---|---|---|---|
| 1200 | ATCACGAGAT | TTCGATTCCA | CCGCCGCCTT | CTATGAAAGG | TTGGGCTTCG |
| 1250 | GAATCGTTTT | CCGGGACGCC | GGCTGGATGA | TCCTCCAGCG | CGGGGATCTC |
| 1300 | ATGCTGGAGT | TCTTCGCCCA | CCCCGGGCTC | GATCCCCTCG | CGAGTTGGTT |
| 1350 | CAGCTGCTGC | CTGAGGCTGG | ACGACCTCGC | GGAGTTCTAC | CGGCAGTGCA |
| 1400 | AATCCGTCGG | CATCCAGGAA | ACCAGCAGCG | GCTATCCGCG | CATCCATGCC |
| 1450 | CCCGAACTGC | AGGAGTGGGG | AGGCACGATG | GCCGCTTTGG | TCGACAATTC |
| 1500 | GCGCTAACTT | ACATTAATTG | CGTTGCGCTC | ACTGCCCGCT | TTCCAGTCGG |
| 1550 | GAAACCTGTC | GTGCCAGCTG | CATTAATGAA | TCGGCCAACG | CGCGGGGAGA |
| 1600 | GGCGGTTTGC | GTATTGGGCG | CCAGGGTGGT | TTTTCTTTTC | ACCAGTGAGA |
| 1650 | CGGGCAACAG | CTGATTGCCC | TTCACCGCCT | GGCCCTGAGA | GAGTTGCAGC |
| 1700 | AAGCGGTCCA | CGCTGGTTTG | CCCCAGCAGG | CGAAAATCCT | GTTTGATGGT |
| 1750 | GGTTAACGGC | GGGATATAAC | ATGAGCTGTC | TTCGGTATCG | TCGTATCCCA |
| 1800 | CTACCGAGAT | ATCCGCACCA | ACGCGCAGCC | CGGACTCGGT | AATGGCGCGC |
| 1850 | ATTGCGCCCA | GCGCCATCTG | ATCGTTGGCA | ACCAGCATCG | CAGTGGGAAC |
| 1900 | GATGCCCTCA | TTCAGCATTT | GCATGGTTTG | TTGAAAACCG | GACATGGCAC |
| 1950 | TCCAGTCGCC | TTCCCGTTCC | GCTATCGGCT | GAATTTGATT | GCGAGTGAGA |
| 2000 | TATTTATGCC | AGCCAGCCAG | ACGCAGACGC | GCCGAGACAG | AACTTAATGG |
| 2050 | GCCCGCTAAC | AGCGCGATTT | GCTGGTGACC | CAATGCGACC | AGATGCTCCA |
| 2100 | CGCCCAGTCG | CGTACCGTCT | TCATGGGAGA | AAATAATACT | GTTGATGGGT |
| 2150 | GTCTGGTCAG | AGACATCAAG | AAATAACGCC | GGAACATTAG | TGCAGGCAGC |
| 2200 | TTCCACAGCA | ATGGCATCCT | GGTCATCCAG | CGGATAGTTA | ATGATCAGCC |
| 2250 | CACTGACGCG | TTGCGCGAGA | AGATTGTGCA | CCGCCGCTTT | ACAGGCTTCG |
| 2300 | ACGCCGCTTC | GTTCTACCAT | CGACACCACC | ACGCTGGCAC | CCAGTTGATC |
| 2350 | GGCGCGAGAT | TTAATCGCCG | CGACAATTTG | CGACGGCGCG | TGCAGGGCCA |
| 2400 | GACTGGAGGT | GGCAACGCCA | ATCAGCAACG | ACTGTTTGCC | CGCCAGTTGT |
| 2450 | TGTGCCACGC | GGTTGGGAAT | GTAATTCAGC | TCCGCCATCG | CCGCTTCCAC |
| 2500 | TTTTTCCCGC | GTTTTCGCAG | AAACGTGGCT | GGCCTGGTTC | ACCACGCGGG |
| 2550 | AAACGGTCTG | ATAAGAGACA | CCGGCATACT | CTGCGACATC | GTATAACGTT |

Fig. 5d

```
          10         20         30         40         50

2600 ACTGGTTTCA CATTCACCAC CCTGAATTGA CTCTCTTCCG GGCGCTATCA
2650 TGCCATACCG CGAAAGGTTT TGCACCATTC GATGGTGTCA ACGTAAATGC
2700 ATGCCGCTTC GCCTTCGCGC GCGAATTGTC GACCCTGTCC CTCCTGTTCA
2750 GCTACTGACG GGGTGGTGCG TAACGGCAAA AGCACCGCCG GACATCAGCG
2800 CTAGCGGAGT GTATACTGGC TTACTATGTT GGCACTGATG AGGGTGTCAG
2850 TGAAGTGCTT CATGTGGCAG GAGAAAAAAG GCTGCACCGG TGCGTCAGCA
2900 GAATATGTGA TACAGGATAT ATTCCGCTTC CTCGCTCACT GACTCGCTAC
2950 GCTCGGTCGT TCGACTGCGG CGAGCGGAAA TGGCTTACGA ACGGGGCGGA
3000 GATTTCCTGG AAGATGCCAG GAAGATACTT AACAGGGAAG TGAGAGGGCC
3050 GCGGCAAAGC CGTTTTTCCA TAGGCTCCGC CCCCCTGACA AGCATCACGA
3100 AATCTGACGC TCAAATCAGT GGTGGCGAAA CCCGACAGGA CTATAAAGAT
3150 ACCAGGCGTT TCCCCTGGCG GCTCCCTCGT GCGCTCTCCT GTTCCTGCCT
3200 TTCGGTTTAC CGGTGTCATT CCGCTGTTAT GGCCGCGTTT GTCTCATTCC
3250 ACGCCTGACA CTCAGTTCCG GGTAGGCAGT TCGCTCCAAG CTGGACTGTA
3300 TGCACGAACC CCCCGTTCAG TCCGACCGCT GCGCCTTATC CGGTAACTAT
3350 CGTCTTGAGT CCAACCCGGA AAGACATGCA AAAGCACCAC TGGCAGCAGC
3400 CACTGGTAAT TGATTTAGAG GAGTTAGTCT TGAAGTCATG CGCCGGTTAA
3450 GGCTAAACTG AAAGGACAAG TTTTGGTGAC TGCGCTCCTC CAAGCCAGTT
3500 ACCTCGGTTC AAAGAGTTGG TAGCTCAGAG AACCTTCGAA AAACCGCCCT
3550 GCAAGGCGGT TTTTTCGTTT TCAGAGCAAG AGATTACGCG CAGACCAAAA
3600 CGATCTCAAG AAGATCATCT TATTAATCAG ATAAAATATT TCTAGATTTC
3650 AGTGCAATTT ATCTCTTCAA ATGTAGCACC TGAAGTCAGC CCCATACGAT
3700 ATAAGTTGTT AATTCTCATG TTTGACAGCT TATCATCGAT
```

Fig. 6

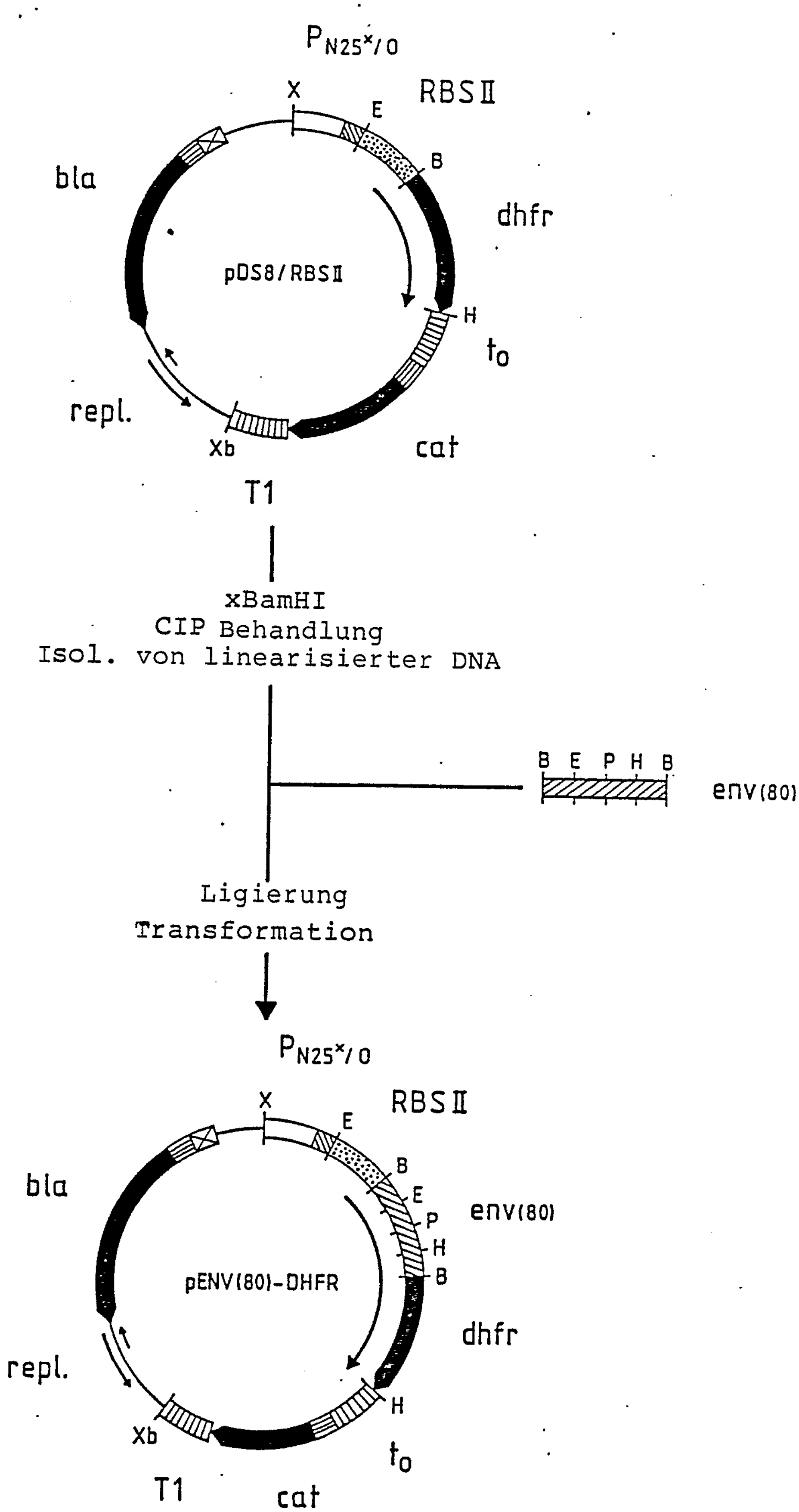
PN25x/0
X
E
RBSII
B
bla
dhfr
pDS8/RBSII
H
t0
repl.
Xb
cat
T1
xBamHI
CIP Behandlung
Isol. von linearisierter DNA
B E P H B
env(80)
Ligierung
Transformation
PN25x/0
X
E
RBSII
B
E
P
H
B
bla
env(80)
pENV(80)-DHFR
dhfr
repl.
H
Xb
t0
T1
cat

Fig. 7a

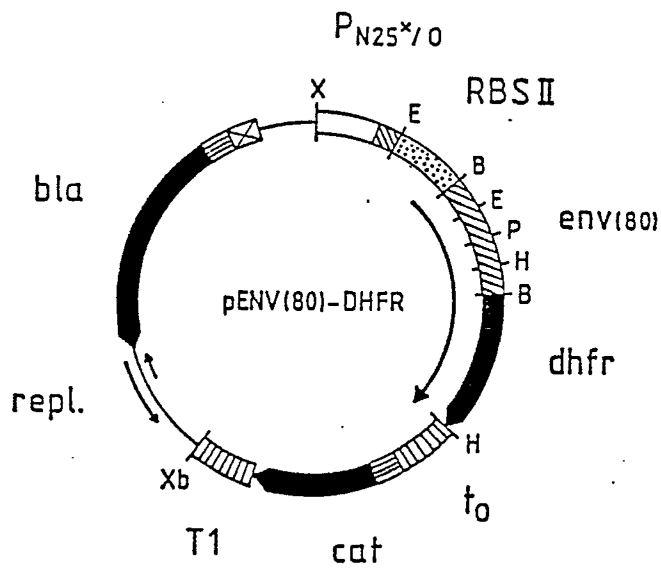


xBamHI Teilverdauung
CIP · Behandlung
xXhoI
Fragment Isolierung

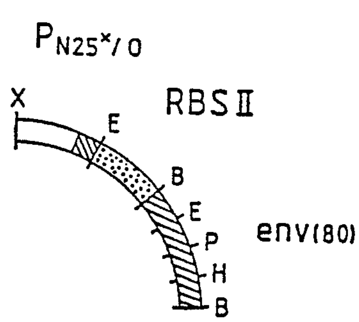


Fragment 1

Fig. 7b

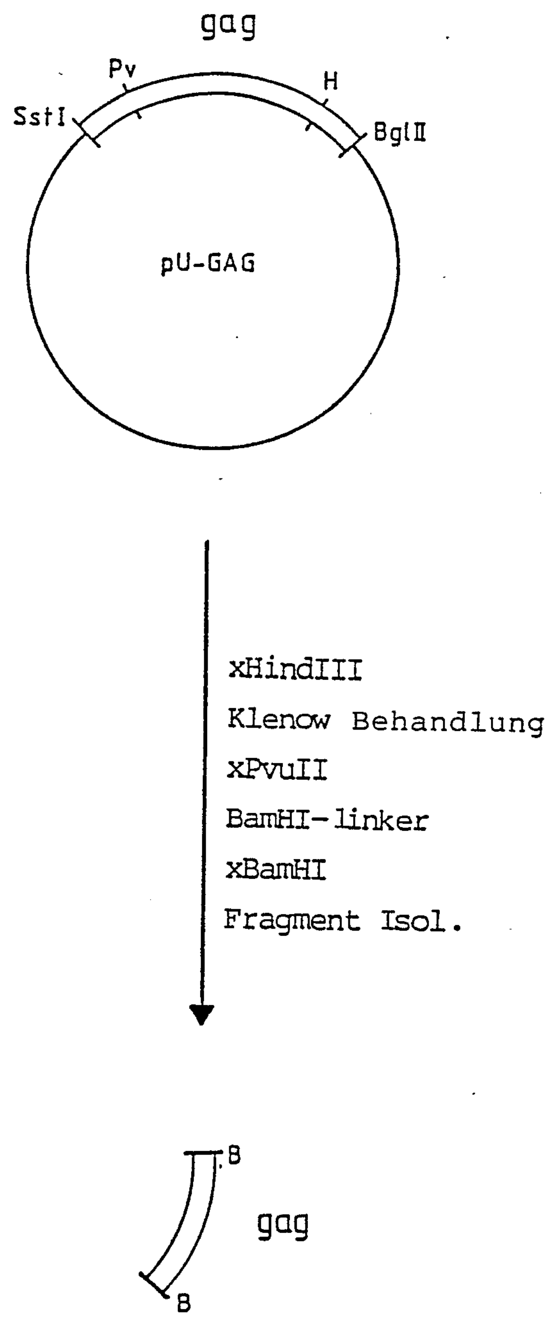


Fragment 2

Fig. 7b (Fortsetzung)

```
                10        20        30        40        50
                 |         |         |         |         |
   1 GAGCTCTCTCGACGCAGGACTCGGCTTGCTGAAGCGCGCACGGCAAGAGG
  51 CGAGGGGGGCGACTGGTGAGTACGCCAAAAATTTTGACTAGCGGAGGCT
 101 AGAAGGAGAGAGATGGGTGCGAGAGCGTCAGTATTAAGCGGGGGAAAATT
 151 AGATCGATGGGAAAAAATTCGGTTAAGGCCAGGGGGAAAGAAAAAATATA
 201 AATTAAAACATATAGTATGGGCAAGCAGGGAGCTAGAACGATTCGCAGTT
 251 AATCCTGGCCTGTTAGAAACATCAGAAGGCTGTAGACAAATACTGGGACA
 301 GCTACAACCATCCCTTCAGACAGGATCAAAAGAACTTAGATCATTATATA
 351 ATACAGTAGCAACCCTCTATTGTGTGCATCAAAGGATAGAGATAAAAGAC
 401 ACCAAGGAAGCTTTAGACAAGATAGAGGAAGAGCAAAACAAAAGTAAGAA
 451 AAAAGCACAGCAAGCAGCAGCTGACACAGGACACAGCAGCCAGGTCAGCC
 501 AAAATTACCCTATAGTGCAGAACATCCAGGGGCAAATGGTACATCAGGCC
 551 ATATCACCTAGAACTTTAAATGCATGGGTAAAAGTAGTAGAAGAGAAGGC
 601 TTTCAGCCCAGAAGTGATACCCATGTTTTCAGCATTATCAGAAGGAGCCA
 651 CCCCACAAGATTTAAACACCATGCTAAACACAGTGGGGGGACATCAAGCA
 701 GCCATGCAAATGTTAAAAGAGACCATCAATGAGGAAGCTGCAGAATGGGA
 751 TAGAGTGCATCCAGTGCATGCAGGGCCTATCGCACCAGGCCAGATGAGAG
 801 AACCAAGGGGAAGTGACATAGCAGGAACTACTAGTACCCTTCAGGAACAA
 851 ATAGGATGGATGACAAATAATCCACCTATCCCAGTAGGAGAAATTTATAA
 901 AAGATGGATAATCCTGGGATTAAATAAAATAGTAAGAATGTATAGCCCTA
 951 CCAGCATTCTGGACATAAGACAAGGACCAAAAGAACCCTTTAGAGACTAT
1001 GTAGACCGGTTCTATAAAACTCTAAGAGCCGAGCAAGCTTCACAGGAGGT
1051 AAAAAATTGGATGACAGAAACCTTGTTGGTCCAAAATGCGAACCCAGATT
1101 GTAAGACTATTTTAAAAGCATTGGGACCAGCGGCTACACTAGAAGAAATG
1151 ATGACAGCATGTCAGGGAGTAGGAGGACCCGGCCATAAGGCAAGAGTTTT
1201 GGCTGAAGCAATGAGCCAAGTAACAAATTCAGCTACCATAATGATGCAGA
1251 GAGGCAATTTTAGGAACCAAAGAAAGATTGTTAAGTGTTTCAATTGTGGC
1301 AAAGAAGGGCACACAGCCAGAAATTGCAGGGCCCCTAGGAAAAAGGGCTG
1351 TTGGAAATGTGGAAAGGAAGGACACCAAATGAAAGATTGTACTGAGAGAC
1401 AGGCTAATTTTTTAGGGAAGATCT
```

Fig. 7c

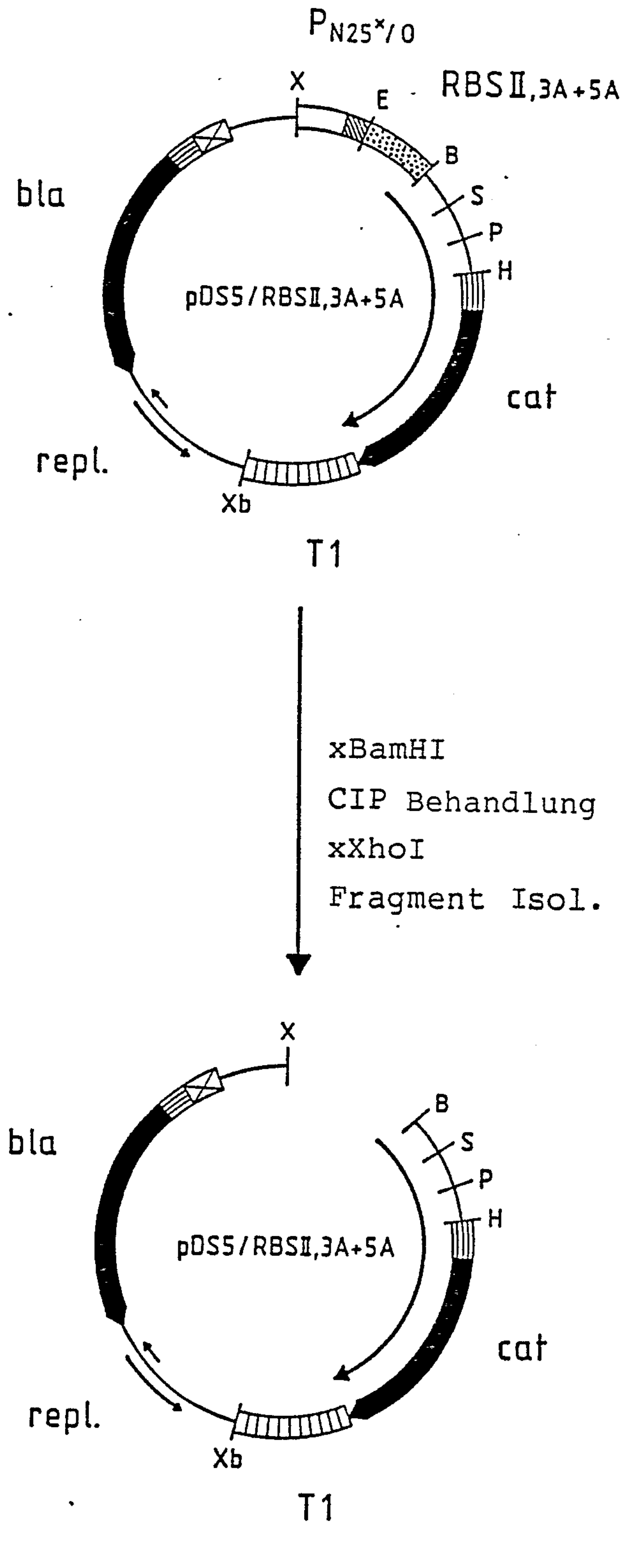


Fragment 3

Fig. 7d

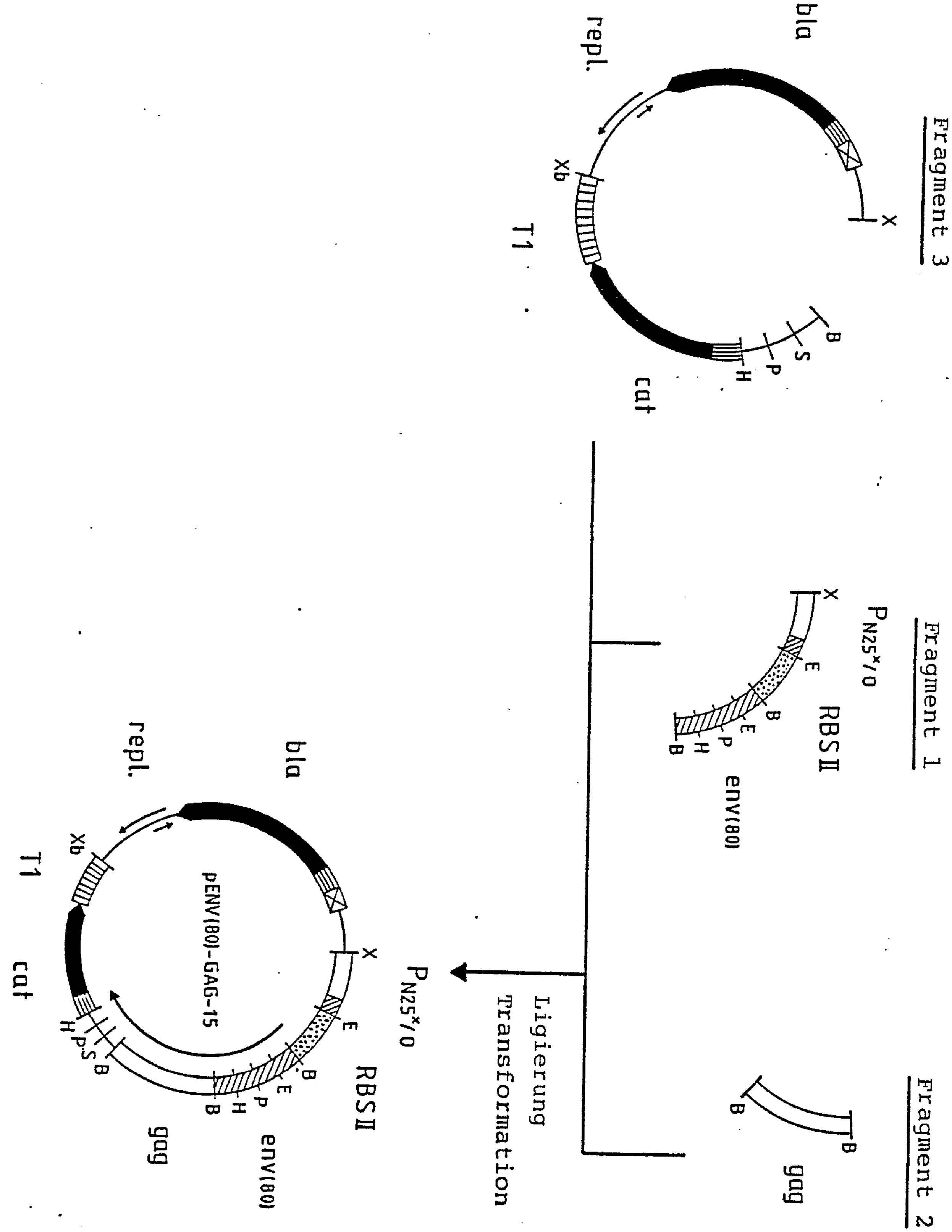
Fragment 3
bla
repl.
Xb
T1
cat
X
B
S
P
H
Fragment 1
$P_{N25x/0}$
RBSII
env(80)
X
E
B
E
P
H
B
Fragment 2
gag
B
B
Ligierung
Transformation
pENV(80)-GAG-15
bla
repl.
Xb
T1
cat
X
E
B
E
P
H
B
S
P
H
RBSII
env(80)
gag

Fig. 8

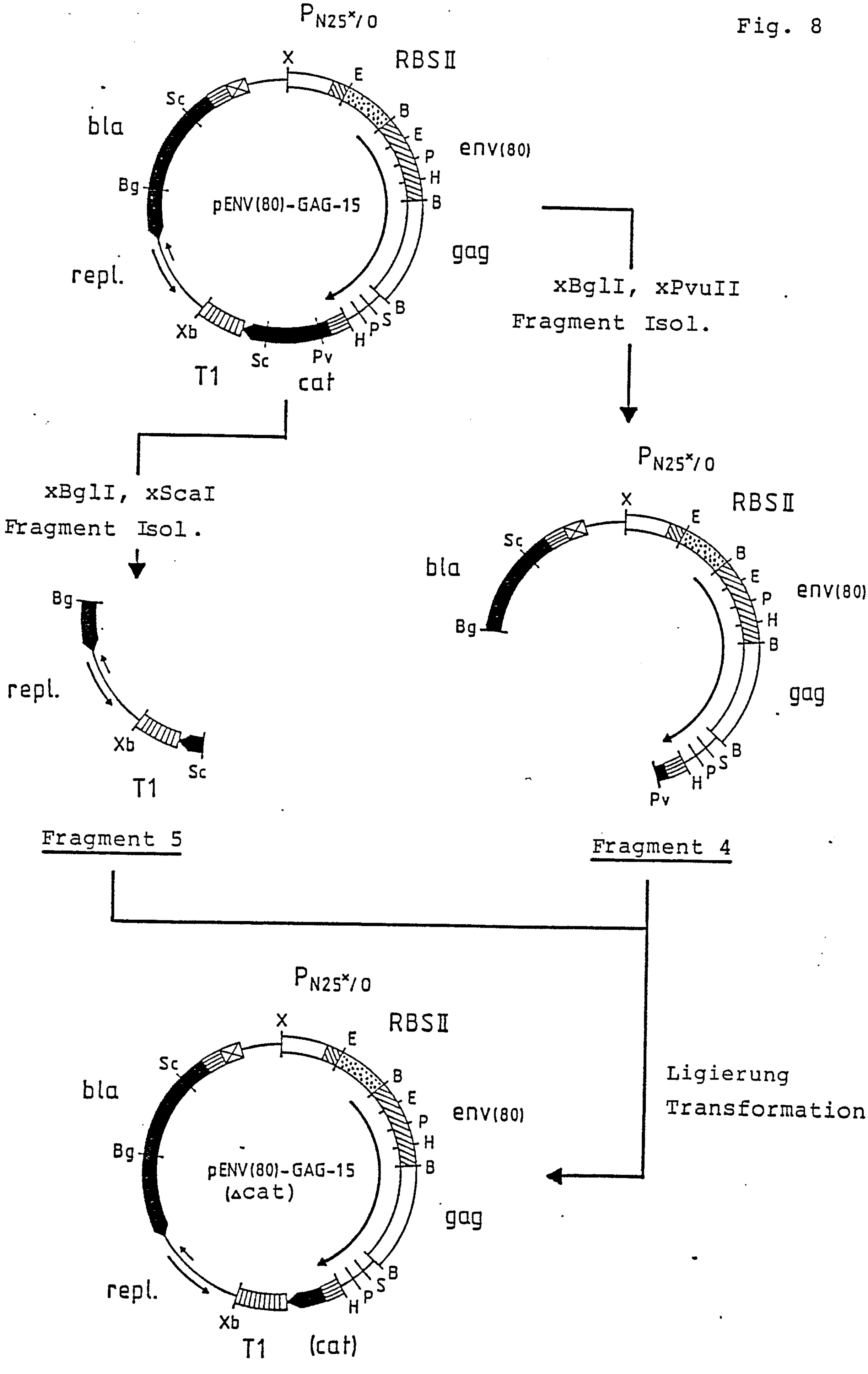

PN25x/0
RBSII
bla
env(80)
gag
repl.
T1
cat
pENV(80)-GAG-15
X
E
B
P
H
S
Sc
Bg
Xb
Pv
xBglI, xPvuII
Fragment Isol.
xBglI, xScaI
Fragment Isol.
Fragment 5
Fragment 4
Ligierung
Transformation
pENV(80)-GAG-15
(Δcat)
(cat)

Fig. 9

10 20 30 40 50 60

ATGAGAGGATCCGAAGCTCAACAGCATCTGCTGCAACTCACTGTTTGGGGTATCAAACAG

<u>MET</u>ArgGlySerGluAlaGlnGlnHisLeuLeuGlnLeuThrValTrpGlyIleLysGln

70 80 90 100 110 120

CTCCAGGCTCGAATTCTGGCTGTTGAACGTTACCTGAAAGATCAACAGCTCCTGGGTATC

LeuGlnAlaArgIleLeuAlaValGluArgTyrLeuLysAspGlnGlnLeuLeuGlyIle

130 140 150 160 170 180

TGGGGCTGCAGTGGTAAACTCATCTGCACTACTGCTGTTCCTTGGAATGCTTCTTGGTCT

TrpGlyCysSerGlyLysLeuIleCysThrThrAlaValProTrpAsnAlaSerTrpSer

190 200 210 220 230 240

AATAAGCTTCTGGAACAGATCTGGAATAACATGACTTGGATGGAGTGGGACCGTGAAATC

AsnLysLeuLeuGluGlnIleTrpAsnAsnMetThrTrpMetGluTrpAspArgGluIle

250 260 270 280 290 300

AACAATTACACTGGATCCGCTGACACAGGACACAGCAGCCAGGTCAGCCAAAATTACCCT

AsnAsnTyrThrGlySerAlaAspThrGlyHisSerSerGlnValSerGlnAsnTyrPro

310 320 330 340 350 360

ATAGTGCAGAACATCCAGGGGCAAATGGTACATCAGGCCATATCACCTAGAACTTTAAAT

IleValGlnAsnIleGlnGlyGlnMetValHisGlnAlaIleSerProArgThrLeuAsn

370 380 390 400 410 420

GCATGGGTAAAAGTAGTAGAAGAGAAGGCTTTCAGCCCAGAAGTGATACCCATGTTTTCA

AlaTrpValLysValValGluGluLysAlaPheSerProGluValIleProMetPheSer

430 440 450 460 470 480

GCATTATCAGAAGGAGCCACCCCACAAGATTTAAACACCATGCTAAACACAGTGGGGGGA

AlaLeuSerGluGlyAlaThrProGlnAspLeuAsnThrMetLeuAsnThrValGlyGly

Fig. 9 (Fortsetzung)

```
        490       500       510       520       530       540
          |         |         |         |         |         |
CATCAAGCAGCCATGCAAATGTTAAAAGAGACCATCAATGAGGAAGCTGCAGAATGGGAT
HisGlnAlaAlaMetGlnMetLeuLysGluThrIleAsnGluGluAlaAlaGluTrpAsp

        550       560       570       580       590       600
          |         |         |         |         |         |
AGAGTGCATCCAGTGCATGCAGGGCCTATCGCACCAGGCCAGATGAGAGAACCAAGGGGA
ArgValHisProValHisAlaGlyProIleAlaProGlyGlnMetArgGluProArgGly

        610       620       630       640       650       660
          |         |         |         |         |         |
AGTGACATAGCAGGAACTACTAGTACCCTTCAGGAACAAATAGGATGGATGACAAATAAT
SerAspIleAlaGlyThrThrSerThrLeuGlnGluGlnIleGlyTrpMetThrAsnAsn

        670       680       690       700       710       720
          |         |         |         |         |         |
CCACCTATCCCAGTAGGAGAAATTTATAAAAGATGGATAATCCTGGGATTAAATAAAATA
ProProIleProValGlyGluIleTyrLysArgTrpIleIleLeuGlyLeuAsnLysIle

        730       740       750       760       770       780
          |         |         |         |         |         |
GTAAGAATGTATAGCCCTACCAGCATTCTGGACATAAGACAAGGACCAAAAGAACCCTTT
ValArgMetTyrSerProThrSerIleLeuAspIleArgGlnGlyProLysGluProPhe

        790       800       810       820       830       840
          |         |         |         |         |         |
AGAGACTATGTAGACCGGTTCTATAAAACTCTAAGAGCCGAGCAAGCTCGGATCCGTCGA
ArgAspTyrValAspArgPheTyrLysThrLeuArgAlaGluGlnAlaArgIleArgArg

        850       860       870
          |         |         |
CCTGCAGCCAAGCTTGGCGAGATTTTCAGGAGCTAA
ProAlaAlaLysLeuGlyGluIlePheArgSer---
```

Fig. 10

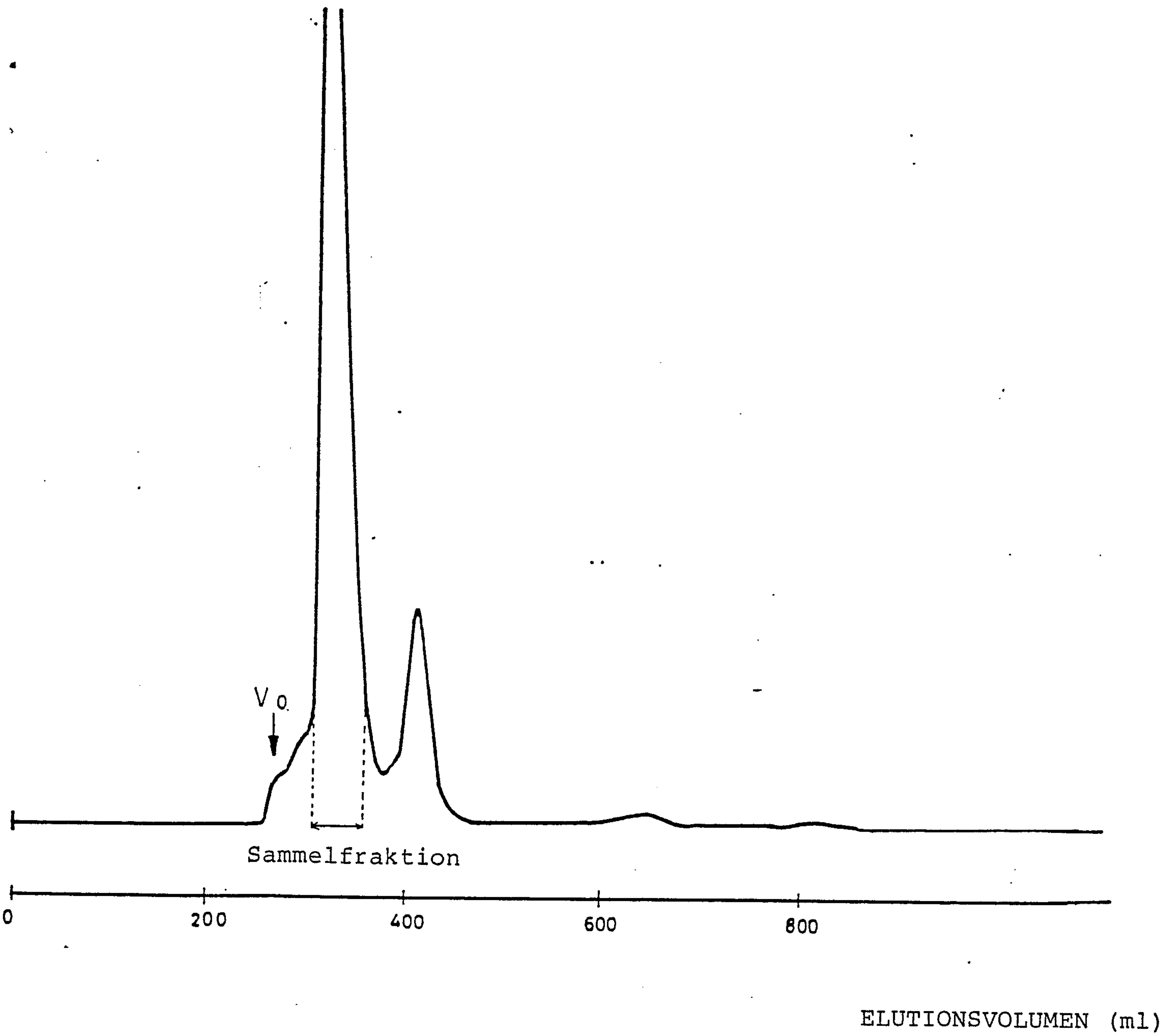

Fig. 11

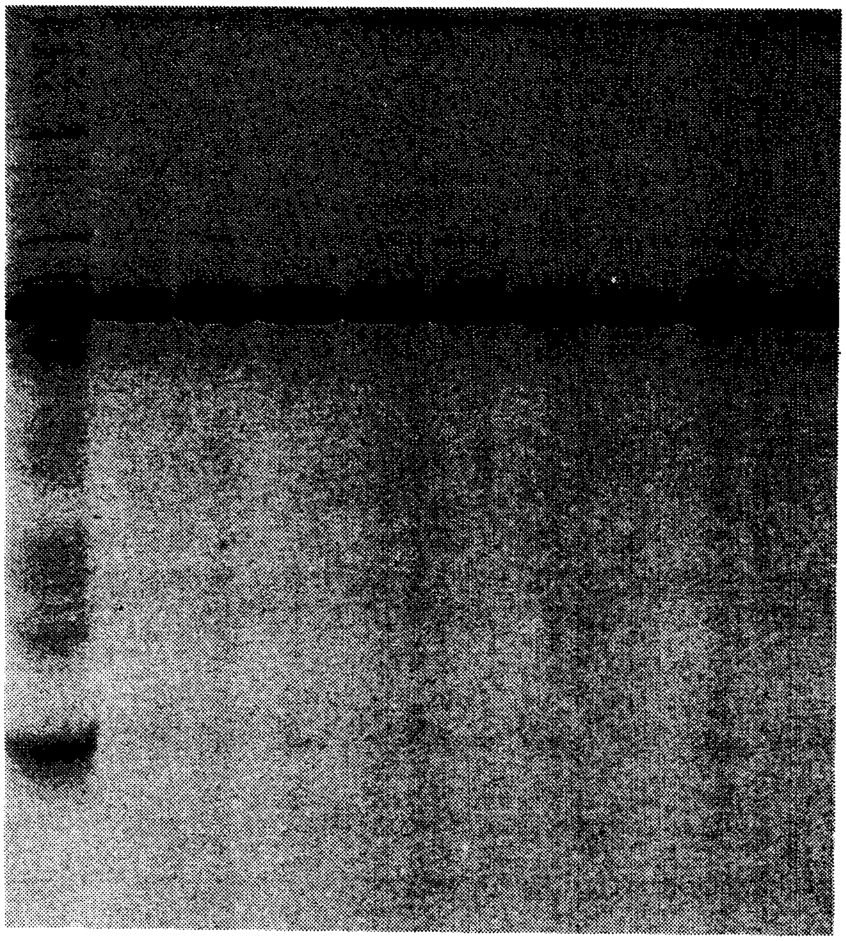

ENV(80)-GAG-15